# EUROPEAN PATENT APPLICATION

(11) **EP 4 427 751 A1**
(43) Date of publication of application: **11.09.2024**
(21) Application number: 22890405.8
(22) Date of filing: 03.11.2022
(51) Int. Cl.: A61K 31/519, A61P 35/00

(54) **PHARMACEUTICAL COMPOSITION COMPRISING PI3K AND DNA-PK DUAL INHIBITOR FOR PREVENTING OR TREATING PERIPHERAL T CELL LYMPHOMA**

(30) Priority: 03.11.2021 KR 20210150125
(71) Applicant: Boryung Corporation, Seoul 03127 (KR)
(72) Inventor: KIM, Bong-Seog, Seoul 06067 (KR); JUNG, Hyo Soon, Seoul 05268 (KR); PARK, Kyoung Ryun, Seongnam-si Gyeonggi-do 13559 (KR); WANG, Seungho, Siheung-si Gyeonggi-do 14988 (KR); JUNG, Seung Hee, Seoul 02222 (KR); KIM, Yoon Sik, Suwon-si Gyeonggi-do 16402 (KR); LEE, Joo Han, Seoul 04199 (KR); JEON, Byeongwook, Incheon 21551 (KR); KIM, Jongyeon, Suwon-si Gyeonggi-do 16452 (KR); KIM, Tae Min, Seoul 03165 (KR); KIM, Seok Jin, Seoul 06647 (KR); YOON, Dok Hyun, Seoul 05507 (KR); JEON, Yoon Kyung, Seoul 06592 (KR); MATTOUR, Ahmad H., Livonia, Michigan 48152 (US); CHAVES, Jorge M., Tacoma, Washington 98465 (US)
(74) Representative: Forrest, Stuart
(86) International application number: PCT/KR2022/017128
(87) International publication number: WO 2023/080674

(57) **Abstract**

The present invention relates to a composition for preventing or treating peripheral T-cell lymphoma, including a compound represented by formula 1 or pharmaceutically acceptable salts thereof. Specifically, the present invention relates to a pharmaceutical composition capable of effectively ameliorating peripheral T-cell lymphoma by decreasing regulatory T cells and increasing CD8+ T cells.

The pharmaceutical composition of the present invention may exhibit anticancer activity against peripheral T-cell lymphoma by decreasing regulatory T cells which suppress immune responses and increasing CD8+ T cells which exhibit anticancer activity. In addition, the pharmaceutical composition of the present invention may be used for the treatment of peripheral T-cell lymphoma due to excellent safety and tolerability.

## Description

### Technical Field

The present invention relates to a composition for preventing or treating peripheral T-cell lymphoma, including a compound represented by formula 1 or pharmaceutically acceptable salts thereof. Specifically, the present invention relates to a pharmaceutical composition capable of effectively ameliorating peripheral T-cell lymphoma by decreasing regulatory T cells and increasing CD8+ T cells.

### Background

Phosphatidylinositol 3-kinase (PI3 kinase; PI3K) is a lipid kinase, which phosphorylates lipid molecules instead of proteins, and plays an important role in cell survival, signal transduction, control of membrane trafficking, etc. If a problem occurs to such control of cell survival, signal transduction, control of membrane trafficking, cancer, inflammatory diseases, autoimmune diseases, etc. occur.

Recently, the results of a study on developing a compound having a novel structure effective in selectively inhibiting PI3 kinase have been reported, and specifically International Publication WO 2016/204429 discloses a compound which has PI3K inhibitory activity and is useful for the treatment of cancer, autoimmune diseases, respiratory diseases and the like, which is incorporated herein by reference. However, a PI3K-δ inhibitor monotherapy is known to have a poor prognosis in non-Hodgkin's lymphoma, which is a malignant lymphoma.

Meanwhile, inhibitors for suppressing multiple PI3K subtypes and/or DNA-PK activation other than PI3K-δ only inhibitors efficiently induce cell cycle arrest and apoptosis, stimulate a decrease in the stability of carcinogens, and thus are expected to exhibit effective anticancer activity in indolent non-Hodgkin's lymphoma, too.

Currently, it is known that about 4,300 cases of non-Hodgkin's lymphoma occur in Korea and approximately 510,000 cases worldwide each year, but therapeutic agents for non-Hodgkin's lymphoma involving multiple inhibitors for suppressing multiple PI3K subtypes and/or DNA-PK activation are not known, and thus there is a need for developing the therapeutic agents.

### Related Art References

### Patent Documents

International Publication WO 2016/204429

### Disclosure Of Invention

### Technical Problem

An object of the present invention is to provide a pharmaceutical composition for preventing or treating peripheral T-cell lymphoma (PTCL), including a compound represented by formula 1 below or pharmaceutically acceptable salts thereof in an amount of 25 mg or more and less than 325 mg as a compound represented by formula 1.

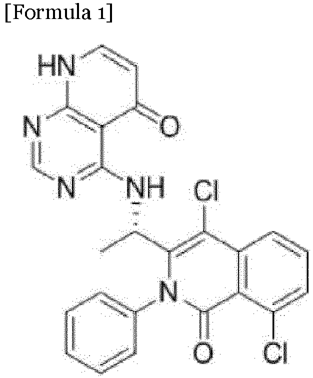

### Technical Solution

The present invention provides a pharmaceutical composition for preventing or treating peripheral T-cell lymphoma (PTCL), including a compound represented by formula 1 below or pharmaceutically acceptable salts thereof in an amount of 25 mg or more and less than 325 mg as the compound.

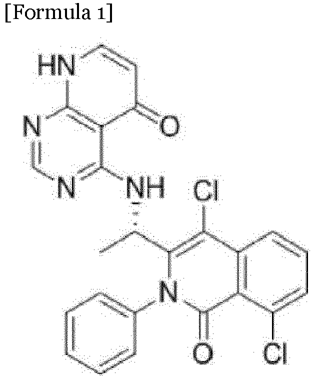

In the present invention, the pharmaceutical composition may be used for preventing or treating peripheral T-cell lymphoma (PTCL), relapsed/refractory B-cell lymphoma, chronic lymphocytic leukemia (CLL)/small lymphocytic leukemia (SLL) other than peripheral T-cell lymphoma (PTCL), and specifically may be used for preventing or treating diffuse large B-cell lymphoma (DLBCL), including MYC-altered diffuse large B-cell lymphoma and transformed diffuse large B-cell lymphoma; follicular lymphoma; chronic Lymphocytic Leukemia (CLL)/small lymphocytic leukemia (SLL); other B-cell lymphomas such as mantle cell lymphoma (MCL), marginal zone lymphoma (MZL), and Waldenstrom macroglobulinemia; and cutaneous T cell lymphoma/mycosis fungoide (composite CTCL-MF) without limitation.

The compound represented by above formula 1 is one of heteroaryl derivatives known to have PI3K and DNA-PK dual inhibitory activity, and is named "4-[[(1S)-1-(4,8-dichloro-1-oxo-2-phenyl-3-isoquinolyl)ethyl]amino]-8H-pyrido[2,3-d]pyrimidin-5-one."

In the present invention, the pharmaceutically acceptable salts mean salts conventionally used in a pharmaceutical industry, for example, inorganic ion salts prepared from calcium, potassium, sodium, magnesium or the like; inorganic acid salts prepared from hydrochloric acid, nitric acid, phosphoric acid, bromic acid, iodic acid, perchloric acid, sulfuric acid or the like; organic acid salts prepared from acetic acid, trifluoroacetic acid, citric acid, maleic acid, succinic acid, oxalic acid, benzoic acid, tartaric acid, fumaric acid, mandelic acid, propionic acid, lactic acid, glycolic acid, gluconic acid, galacturonic acid, glutamic acid, glutaric acid, glucuronic acid, aspartic acid, ascorbic acid, carbonic acid, vanillic acid or the like; sulfonic acid salts prepared from methanesulfonic acid, ethanesulfonic acid, benzenesulfonic acid, p-toluenesulfonic acid, naphthalenesulfonic acid or the like; amino acid salts prepared from glycine, arginine, lysine, etc.; amine salts prepared from trimethylamine, triethylamine, ammonia, pyridine, picoline, etc.; or the like, but types of salt meant in the present invention are not limited to those listed salts.

In the present invention, the composition may include the compound represented by above formula 1 or pharmaceutically acceptable salts thereof as the compound in an amount of 25 mg or more and less than 325 mg, 50 mg or more and less than 325 mg, 100 mg or more and less than 325 mg, or 200 mg or more and less than 325 mg, but is not limited thereto.

In one embodiment of the present invention, the composition may include the compound represented by above formula 1 or pharmaceutically acceptable salts thereof as the compound in an amount of 50 mg, 100 mg or 200 mg, preferably 200 mg.

When administering the composition to a subject with peripheral T-cell lymphoma (PTCL) according to the present invention, a maximum tolerated dose (MTD) of the compound represented by above formula 1 may be 200 mg QD, and have excellent tolerability and safety in this dose range. In addition, based on the tolerability and safety, 200 mg QD of the compound represented by above formula 1 may be recommended as a starting dose (RP2D) for clinical phase Ib/II.

The above dose range in the composition according to the present invention may have safety and tolerability in subjects with peripheral T-cell lymphoma (PTCL), and thus may be used for the treatment of subjects diagnosed with peripheral T-cell lymphoma (PTCL).

In the composition according to the present invention, the pharmaceutically acceptable salt of the compound represented by above formula 1 may mean the compound represented by above formula 1 which is included in an amount of 25 mg or more and less than 325 mg, 50 mg or more and less than 325 mg, 100 mg or more and less than 325 mg, and 200 mg or more and less than 325 mg but is not limited thereto, preferably 25 mg, 50 mg, 100 mg or 200 mg, and more preferably 200 mg.

The present invention may effectively prevent or treat peripheral T-cell lymphoma (PTCL) by showing excellent safety and tolerability in subjects with peripheral T-cell lymphoma (PTCL) in the above dose range.

In the present invention, the pharmaceutical composition may be administered to a subject in need thereof in an amount of 25 mg or more and less than 325 mg, 50 mg or more and less than 325 mg, 100 mg or more and less than 325 mg, 200 mg or more and less than 325 mg, preferably 25 mg, 50 mg, too mg or 200 mg once daily (QD) for 28 consecutive days (cycle 1, four weeks) or more.

For example, subjects who have completed the first cycle may continue to receive a test drug at the same dose as in cycle 1 until the occurrence of tumor progression, toxicity exceeding a tolerability limit, or withdrawal of consent. For example, the subjects may receive the test drug for at least 4, at least 8, at least 12, at least 16, at least 20, at least 24, at least 28, at least 32, or at least 36 weeks, but are not limited thereto.

In the present invention, if emerging new safety, tolerability, and PK data indicate that a more frequent dosing regimen would be appropriate, a dosing regimen such as once daily (QD) may be changed to twice daily (BID), but is not limited thereto. In addition, the dose of the compound represented by above formula 1 according to the present invention may be subject to change taking a total daily exposure of the new regimen (area under the concentration-time curve, AUC) into account, as long as the dose does not exceed the AUC established as safe and tolerated after QD administration.

In the present invention, the pharmaceutical composition may be administered to a subject diagnosed with peripheral T-cell lymphoma (PTCL), specifically, a subject who has received at least one chemotherapy after being diagnosed with peripheral T-cell lymphoma (PTCL). The chemotherapy may refer to a systemic treatment method which prevents cancer cells from growing or kills the same by using a drug (anticancer agent), which is a standard treatment method for cancer along with surgery and radiation therapy, and may include anthracycline-based chemotherapy (regimens), rituximab and/or nivolumab-based immunotherapy, but is not limited thereto.

In the present invention, the "subject" may mean mammals including humans, and specifically may mean humans.

In the present invention, the above pharmaceutical composition may be orally or parenterally administered (for example, intravenously, subcutaneously, intraperitoneally or locally administered), and a dose thereof may be appropriately changed depending on an administration route. In one embodiment of the present invention, the composition may be orally administered.

In the present invention, the term "oral administration" may mean one, in which a substance prepared for allowing an active substance to be digested is administered into the gastrointestinal tract for absorption. A non-limiting example of the preparation for oral administration may include tablets, troches, lozenges, water soluble suspensions, oil suspensions, prepared powders, granules, emulsions, hard capsules, soft capsules, syrups, elixirs or the like. To formulate the pharmaceutical composition of the present invention into a preparation for oral administration, the followings may be used: binders such as lactose, saccharose, sorbitol, mannitol, starch, amylopectin, cellulose, gelatin or the like; diluents such as dicalcium phosphate, etc.; disintegrants such as maize starch, sweet potato starch or the like; lubricants such as magnesium stearate, calcium stearate, sodium stearyl fumarate, polyethylene glycol wax, or the like; etc., in which sweetening agents, flavoring agents, syrups, etc. may also be used. Furthermore, in case of the capsules, liquid carriers such as fatty oil, etc. may be further used in addition to the above-mentioned materials.

The pharmaceutical composition may be prepared in the form of oral or parenteral preparation, which is apparent to those skilled in the art. In one embodiment of the present invention, the pharmaceutical composition may be a preparation for oral administration. In the present invention, the above preparation for oral administration may be a solid preparation such as a tablet, pill, powder, granule, capsule, etc. or a liquid preparation such as a suspension, liquid for internal use, emulsion, syrup, etc., preferably the solid preparation, more preferably the capsule.

If the pharmaceutical composition of the present invention is formulated into an oral solid preparation, the examples of the additives used herein may include cellulose, calcium silicate, corn starch, lactose, sucrose, dextrose, calcium phosphate, stearic acid, magnesium stearate, calcium stearate, gelatin, talc, etc.

If the pharmaceutical composition is formulated into a liquid preparation for oral administration, the pharmaceutical composition may be formulated by adding simple diluents such as water, liquid or paraffin, and various additives such as a wetting agent, sweetener, fragrance, preservative, antiseptic, colorant, etc.

When the pharmaceutical composition of the present invention is prepared in the form of an injection, the additives may include water, saline, aqueous glucose solution, pseudo-sugar aqueous solution, alcohol, glycol, ether (e.g., polyethylene glycol 400), oil, fatty acid, fatty acid ester, glyceride, etc.

In the present invention, the pharmaceutical composition may decrease regulatory T cells (T_{regs}) and increase CD8+ T cells. The pharmaceutical composition may exhibit excellent anticancer activity against advanced hematologic cancer by decreasing regulatory T cells which suppress immune responses and increasing CD8+ T cells which exhibit anticancer effects. In one embodiment of the present invention, the advanced hematologic cancer may be peripheral T-cell lymphoma (PTCL).

In the present invention, the pharmaceutical composition may further include an active ingredient which has pharmacological activity other than the compound represented by above formula 1 or pharmaceutically acceptable salts thereof.

In the present invention, the pharmaceutical composition may further include a pharmaceutically acceptable additive. As used herein, the "pharmaceutically acceptable" may mean one which is physiologically acceptable; which does not conventionally cause an allergic reaction such as gastrointestinal disturbance and dizziness, or other similar reactions thereto, when being administered into a human; and which is conventionally used by those skilled in the art in preparing a pharmaceutical composition with reference to Remington's Pharmaceutical Science (latest version), Mack Publishing Company, Easton PA.

The above additive may be a carrier, diluent, extender, antioxidant, buffer, filler, anticoagulant, lubricant, humectant, a flavoring agent, emulsifier, a suspending agent, surfactant, antiseptic, etc. For example, the above additive may be lactose, dextrose, calcium silicate, maize starch, sucrose, sorbitol, mannitol, xylitol, erythritol, maltitol, starch, acacia rubber, alginate, gelatin, calcium phosphate, calcium silicate, cellulose, methyl cellulose, polyvinyl pyrrolidone, water, methyl hydroxybenzoate, propyl hydroxybenzoate, talc, stearic acid, magnesium stearate, calcium stearate, mineral oil, saline solution, glucose aqueous solution, similar sugar aqueous solution, alcohol, glycol, ether (ex: polyethylene glycol 400), oil, fatty acid, fatty acid ester, glyceride or mixtures thereof. However, additives which may be contained in the composition according to the present invention are not limited to those listed substances, which are intended as examples only. In the present invention, a content of additives contained in the pharmaceutical composition is not particularly limited, and may be appropriately adjusted within a range of contents conventionally used in formulating a preparation.

In the present invention, the term "prevention" may refer to an inhibition or a delay of occurrence of disease, disorder or condition. If the occurrence of disease, disorder or condition is inhibited or delayed for an expected period of time, the prevention may be considered as complete.

In the present invention, the term "treatment" may refer to one which partially or completely reduces, ameliorates, alleviates, inhibits or delays a certain disease, disorder and/or condition, or symptoms of the condition, reduces severity thereof, or reduces the occurrence of at least one symptom or property thereof.

The present invention may provide a use of a pharmaceutical composition for preventing or treating advanced hematologic cancer, which includes a compound represented by formula 1 below or pharmaceutically acceptable salts thereof in an amount of 25 mg or more and less than 325 mg as a compound represented by formula 1. In one embodiment of the present invention, the advanced hematologic cancer may be peripheral T-cell lymphoma (PTCL).

The present invention may provide a use of a pharmaceutical composition in the manufacture of a medication for preventing or treating advanced hematologic cancer, which includes a compound represented by formula 1 below or pharmaceutically acceptable salts thereof in an amount of 25 mg or more and less than 325 mg as a compound represented by formula 1. In one embodiment of the present invention, the advanced hematologic cancer may be peripheral T-cell lymphoma (PTCL).

The present invention may provide a method for preventing or treating advanced hematologic cancer by administering into a subject a pharmaceutical composition which includes a compound represented by formula 1 below or pharmaceutically acceptable salts thereof in an amount of 25 mg or more and less than 325 mg as a compound represented by formula 1. In one embodiment of the present invention, the advanced hematologic cancer may be peripheral T-cell lymphoma (PTCL).

Matters mentioned in the pharmaceutical composition of the present invention, the treatment method thereof and the use thereof are applied the same, if not contradictory to each other.

### Advantageous Effects

The pharmaceutical composition of the present invention may have excellent safety and tolerability in the treatment of peripheral T-cell lymphoma.

In addition, the pharmaceutical composition of the present invention may effectively treat peripheral T-cell lymphoma by decreasing regulatory T cells which suppress immune responses and by increasing CD8+ T cells which exhibit anticancer effects.

### Brief Description of the Drawings

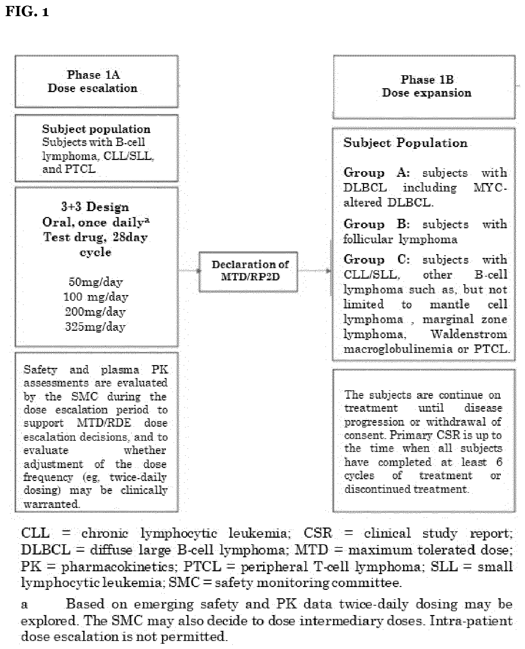

FIG. 1 is a schematic view showing a clinical trial phase I design.
FIG. 2 is a schematic view showing a dose escalation trial design (cohorts 1 and 2).
FIG. 3 is a schematic view showing a dose escalation trial design (cohorts 3 and subsequent).
FIG. 4 shows the results of pharmacokinetic (PK) analyses of plasma samples on day 1 of cycle 1 (C1D1) after administration of a test drug to a subject.
FIG. 5 shows the results of pharmacokinetic (PK) analyses of plasma samples on day 15 of cycle 1 (C1D15) after administration of a test drug to a subject.
FIG. 6a is a scatter plot of a PK-PD correlation of changes in regulatory T cell expression after administration of a test drug.
FIG. 6b is a scatter plot of a PK-PD correlation of changes in CD8+ T cell expression after administration of a test drug.

### Mode for Invention

Hereinafter, the present invention will be described in detail through Examples for better understanding of the present invention. However, the following Examples are provided only for the purpose of illustrating the present invention, and thus the scope of the present invention is not limited thereto. The Examples of the present invention are provided to more completely describe the present invention to those having ordinary skill in the art.

### Example

### 1. Subjects inclusion

A total of 12 subjects with advanced hematologic malignancies were included for studying of phase Ia of this clinical trial.

The above subjects included those who have pathological subtypes of peripheral T-cell lymphoma (PTCL) (n = 8), diffuse large B-cell lymphoma (DLBCL) (n = 2), marginal zone lymphoma (MZL) (n = 1), and cutaneous T-cell lymphoma/mycosis fungoides (composite CTCL-MF) (n = 1).

The demographic and other underlying characteristics of the above subjects are as follows:
- Male (n = 9) and female (n = 3)
- Median age (63, range 30-76)
- ECOG PS (0-1)
- All the subjects have previously experienced at least one chemotherapy.

### A. Inclusion criteria

The subjects are considered to be eligible for participating in the clinical trial only if they meet all of the following criteria:
1) Be capable of giving signed informed consent. This also includes complying with the requirements and restrictions listed in the informed consent form (ICF) and in the protocol.
2) Male or female subjects 18 years of age or older (If a legal age of consent to participate in clinical trials is > 18 years old, local regulatory requirements need to be followed.)
3) ECOG performance status ≤ 2.
4) Life expectancy in excess of 3 months.
5) Phase Ia (dose escalation part only):
   Those who have received a standard therapy or are intolerant of standard therapy, and/or for whom no standard therapy exists, while being diagnosed with relapsed and/or refractory B-cell lymphoma, chronic lymphocytic leukemia (CLL)/small lymphocytic leukemia (SLL) and peripheral T-cell lymphoma (PTCL) according to the World Health Organization (WHO) classification.
6) Phase Ib (dose expansion only):
   Those who have received a standard therapy or are intolerant of standard therapy, or for whom no standard therapy exists and fit into one of the following groups, while being diagnosed with relapsed and/or refractory lymphoma (according to the World Health Organization (WHO) classification).
   - Group A: Subjects who are diagnosed with diffuse large B-cell lymphoma (DCBCL), including MYC-altered DLBCL and transformed DLBCL.
   - Group B: Subjects who are diagnosed with grade 1 to 3a follicular lymphoma.
   - Group C: Subjects who are diagnosed with CLL/SLL and other B-cell lymphomas (e.g., including, but not limited to, mantle cell lymphoma (MCL), marginal zone lymphoma (MZL), Waldenstrom macroglobulinemia, or peripheral T-cell lymphoma (PTCL)).
7) All the subjects enrolled in phases Ia and Ib (dose expansion) should have measurable disease based on the appropriate tumor type criteria.
8) Subjects currently in need of systemic therapy according to an investigator's assessment.
9) An archival or fresh tumor tissue (ie, tissue block or series of approximately 15 slides) is required and should be provided during the screening visit for lymphoma subjects. For CLL and Waldenstrom macroglobulinemia, this is not required if no available tumor tissue.
10) the first 7 subjects in each group of Phase Ia and Phase Ib should be prepared to undergo a fresh tumor biopsy during a clinical trial period. For the CLL and Waldenstrom macroglobulinemia, the biopsy is not mandatory. For subjects with disease sites unsuitable for biopsy, exceptions may be considered after discussion with a sponsor.
11) Female subjects must be surgically sterile, or have a monogamous partner who is surgically sterile, or be of postmenopausal status (at least 1 year amenorrhea or serum follicle-stimulating hormone > 40 mIU/mL and estradiol < 20 pg/mL or a condition as defined to be in a "postmenopausal" range for related trials), or commit to use an effective form of birth control for the duration of clinical trial and for 3 months after the last test drug administration.
12) Sexually active males must commit to use an effective form of birth control during the period of taking the test drug and for 3 months after stopping the administration of the test drug. A condom is required to be used by vasectomized men to prevent delivery of the drug via seminal fluid.
13) Those who represent laboratory test values defined as follows:
   - Creatinine clearance (calculated using the Cockcroft-Gault formula, or measured) > 60 mL/min.
   - Total bilirubin < 1.5 × ULN (except for subjects with Gilbert's syndrome who are excluded if total bilirubin > 3.0 × ULN or direct bilirubin < 1.5 × ULN).
   - ALT <2.5 × ULN (except for subjects who have tumor involvement of the liver, who are included if ALT < 5 × ULN).
   - Absolute neutrophil count (ANC) > 1.0 × 10⁹/L
   - Platelet count > 75 × 10⁹/L
   - Hemoglobin > 8 g/dL

### B. Exclusion criteria

Subjects who meet any one of the following criteria are excluded from this clinical trial.
1) Presence of overt leptomeningeal or active central nervous system (CNS) metastases, or CNS metastases that require local CNS-directed therapy (e.g., radiotherapy or surgery) or increasing doses of corticosteroids within the prior 2 weeks. Brain metastasis subjects who received treatment need to be in a neurologically stable state (for four weeks after administration and before enrollment into clinical trial), and off steroids for at least two weeks before administration of the test drug.
2) Impaired cardiac function or clinically significant cardiac disease, including any of the following:
   - Clinically significant and/or uncontrolled heart disease such as congestive heart failure requiring treatment (New York Heart Association Grade ≥ 2), left ventricular ejection fraction < 50% as determined by multiple gated acquisition (MUGA) or echocardiogram (ECHO), uncontrolled hypertension, or clinically significant arrhythmia.
   - QT interval corrected for heart rate using Fridericia's formula (QTcF) > 450 ms ECG or congenital long QT syndrome at the Screening Visit. If the QTcF cannot be calculated appropriately due to underlying conditions such as Left Bundle Branch Block (LBBB), the eligibility of the subject will be discussed with the medical monitor and Sponsor. The decision of eligibility will be documented appropriately.
   - A marked baseline prolongation of QT/QTc interval (e.g., repeated demonstration of a QTc interval > 450 ms).
   - The obligatory use of concomitant medications that prolong the QT/QTc interval.
   - Acute myocardial infarction or unstable angina pectoris < 3 months prior to clinical trial entry.
3) Subjects with a history of interstitial pneumonia or drug-induced interstitial pneumonia/pneumonitis.
4) Human immunodeficiency virus (HIV) infection.
5) Subjects who are positive for hepatitis B surface antigen (HBsAg) or hepatitis C virus antibody. If negative for HbsAg and positive for the anti-hepatitis B core antibody, a hepatitis B virus-deoxyribonucleic acid (DNA) viral load should not be detected in order to be eligible to participate in the clinical trial.
6) Subjects with chronic liver disease or chronic hepatitis (Child-Pugh class B or C hepatic impairment).
7) Any gastrointestinal disorders which interferes with the absorption of the test drug or makes it impossible to swallow tablets or capsules.
8) Malignant disease, other than that being treated in this clinical trial. Exceptions to this exclusion criteria include the following:
   - malignancies that were treated curatively and have not recurred within 2 years prior to administration of the test drug.
   - completely resected basal cell and squamous cell skin cancers.
   - any malignancy considered to be indolent and that has never required therapy.
   - completely resected carcinoma in situ of any type.
9) Prior PI3K inhibitor is accepted in the dose escalation part of the study (Phase Ia) only.
10) For subjects with lymphoma:
   - Systemic antineoplastic therapy (including cytotoxic chemotherapy, α-interferon [INF], and toxin immunoconjugates) or any experimental therapy within 3 weeks or 5 half-lives, whichever is shorter, before administration of a first dose of the test drug.
   - Therapy with tyrosine kinase inhibitor within 5 half-lives before administration of the first dose of the test drug.
   - Unconjugated monoclonal antibody therapies < 6 weeks before administration of the first dose of the test drug.
11) For subjects receiving a systemic chronic steroid therapy or any immunosuppressive therapy (> 10 mg/day prednisone or equivalent). Topical, inhaled, nasal, and ophthalmic steroids are allowed.
12) Use of any live vaccines against infectious diseases within 4 weeks of test drug administration.
13) Use of hematopoietic colony-stimulating growth factor (e.g., granulocyte colony stimulating factor (GCSF), granulocyte-macrophage colony stimulating factor (MCSF)), macrophage colony stimulating factor (MCSF)), thrombopoietin mimetics, or erythroid-stimulating agents ≤ 2 weeks prior to the start date of administration of the test drug. If the administration of an erythrocyte stimulant has been started at least two weeks before a day of first dosing of the test drug and subjects are on a stable dose, the stimulant may be continuously used.
14) Subjects with a history of stroke or having active neurological symptoms (with the exception of chronic conditions which, in the opinion of the neurologist, Investigator, and the Sponsor, would not impact ongoing neurologic assessments while on clinical trial).
15) Active infections requiring a systemic therapy or an antiviral antibiotic therapy.
16) Subjects with active cytomegalovirus (CMV) infection.
17) Subjects receiving moderate or potent CYP3A4 inhibitors or inducers and are unable to withdraw until 2 weeks or 5 times longer than the half-life, whichever is shorter, before the first dose of test drug treatment.
18) Major surgery performed within two weeks before a day of first dosing of the test drug (mediastinoscopy, insertion of a central venous access device, and insertion of a feeding tube are not considered major surgery).
19) Radiotherapy within 2 weeks of first dose of the test drug, except for palliative radiotherapy to a limited-field, such as for the treatment of bone pain or a focally painful tumor mass.
20) Presence of CTCAE ≥ Grade 2 toxicity (except alopecia, peripheral neuropathy, and ototoxicity, which are excluded if ≥ CTCAE Grade 3) due to prior cancer therapy.
21) Participation in an interventional, investigational study within 2 weeks or 5 half-lives, whichever is shorter, of the first dosing of the test drug.
22) Any medical condition that would, in the investigator's judgment, prevent the subject's participation in the clinical trial due to safety concerns, compliance with clinical trial procedures, or interpretation of test results.
23) Pregnant or lactating women, where pregnancy is defined as the state of a female after conception and until the termination of gestation, confirmed by a positive human chorionic gonadotropin laboratory test.

### 2. Clinical trial design

### A. Test drug

The test drug is 4-[[(1S)-1-(4,8-dichloro-1-oxo-2-phenyl-3-isoquinolyl)ethyl]amino]-8H-pyrido[2,3-d]pyrimidin-5-one (Table 1).

**[Table 1]**

| | **Test drug** |
|---|---|
| **IUPAC name** | 4-[[(1S)-1-(4,8-dichloro-1-oxo-2-phenyl-3-isoquinolyl)ethyl]amino]-8H-pyrido[2,3 -d]pyrimidin-5-one |
| **Dosage formulation** | White hard capsules (white on both the top and the bottom) containing white to pale yellow powder |
| **Manufacturer** | Boryung Pharmaceutical Co., Ltd. |

### B. Dosage and dosing regimen

A total of 12 subjects enrolled into the clinical trial were orally administered with the test drug once daily for a 28-day cycle (open-label, multicenter) (Table 2).

**[Table 2]**

| **Cohort no. (# of subjects)** | **Proposed Daily Dose of test drug (QD)** |
|---|---|
| **Cohort 1 (1)** | 50 mg |
| **Cohort 2 (1)** | 100 mg |
| **Cohort 3 (3)** | 200 mg |
| **Cohort 4 (4)** | 325 mg |
| **Cohort 5 (3)** | 200 mg (Dose expansion) |

In this clinical trial, a starting dose (50 mg QD) of the test drug was determined based on the results of preclinical tests on animals (safety, tolerability and pharmacokinetic data) and toxicity and efficacy obtained from drugs with a mechanism similar to that of PI3K-δ inhibitors in clinical trials.

The dosing regimen used in this clinical trial may be changed if it is judged that more frequent administration, such as BID, is appropriate in consideration of new safety, tolerability, and pharmacokinetic data. In addition, the dosage of this clinical trial may be changed in consideration of a total daily exposure of a new regimen (area under the concentration-time curve, AUC), as long as the dosage does not exceed the established AUC to be safe and tolerable after QD administration (Table 3).

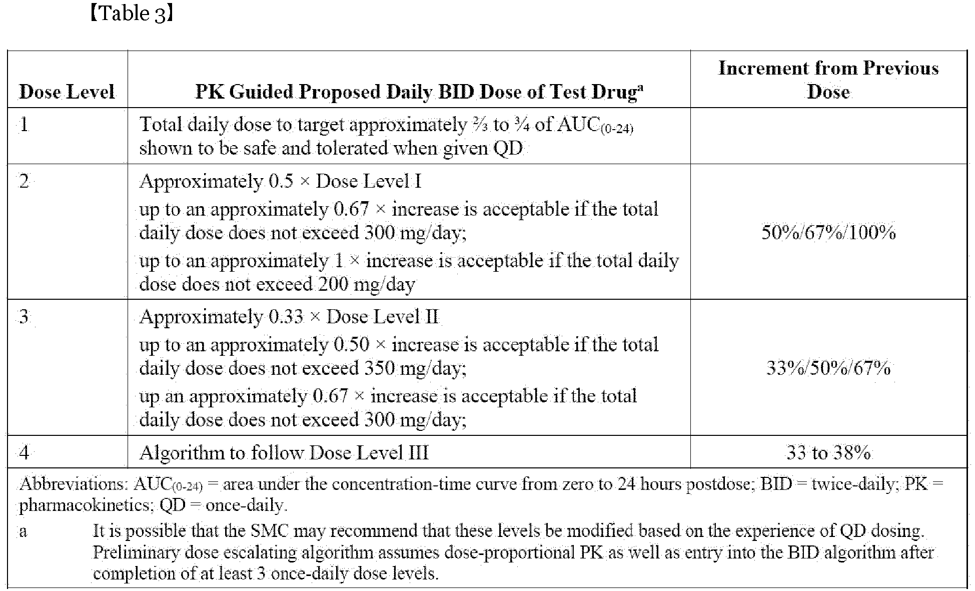

Precautions for administering the test drug are as follows:
1) Subjects need to take the test drug at least two hours before meals or two hours after meals according to the investigator's instructions.
2) If vomiting occurs during administration, no re-dosing of the subject is allowed before the next scheduled dose.
3) For the QD regimen, if the subject forgets to take his/her dose and can take the dose within 12 hours of the usual time of administration, the dose can be taken. If not, then the dose should be withheld that day and restarted on the next scheduled day. For the BID regimen, if applicable, the dose can only be taken within 6 hours of the usual time of administration.
4) On days when serial blood samples are to be collected for PK profile analyses Cycle 1 Days 1 and 15), subjects are instructed to take the test drug in a fasting state (no food or water after midnight) and refrain from eating for 2 hours following the test drug administration. After this period, food intake is allowed.
5) On the day of PET scans, the subject is required to fast from 6 hours before the PET scan but will be allowed water ad libitum. If a test drug dose is planned on that day the following will apply:
   - If a scan is scheduled in the morning, the test drug may be taken with water, but the subjects are required not to eat breakfast (usually following the instructions of the investigator).
   - If the PET scan is scheduled for the afternoon, the subjects may have a light breakfast 4 hours (or more) before the PET scan appointment and take the test drug dose with water 4 hours after the light breakfast. After taking the dose of test drug, the subjects will be required to continue to fast until the PET scan is completed.

### 3. Overall design

This phase I clinical trial is a multicenter, open-label, and first-in-human (FIH) study consisting of two trial parts (phases Ia and Ib).

The phase Ia part (dose escalation) of this clinical trial is designed to determine safety, tolerability, and maximum tolerated dose (MTD)/starting dose (RP2D) for clinical phase Ib/II of the test drug in subjects with relapsed/refractory B-cell lymphoma, chronic lymphocytic leukemia (CLL)/small lymphocytic leukemia (SLL) and peripheral T-cell lymphoma (PTCL).

The phase Ib part (dose expansion) of this clinical trial is designed to assess a tumor response and safety in specific advanced relapsed/refractory B-cell lymphoma, chronic lymphocytic leukemia (CLL)/small lymphocytic leukemia (SLL) and peripheral T-cell lymphoma (PTCL), at a dose of the test drug identified in phase Ia part. Once the recommended dose for expansion (RDE) has been determined in phase Ia (dose escalation), the phase Ib part (dose escalation) commence.

A planned total trial period for each subject enrolled into the entire clinical trial is approximately 10 months. For subjects without disease progression, the trial period may be extended and the entire clinical trial consists of a screening period, a test drug administration period, and a follow-up period. The details of each period are as follows.

### A. Screening period

Informed consent was obtained prior to any clinical trial-related procedures or evaluations. After signing the informed consent, all potential subjects were screened (Days -21 to -1).

### B. Test drug treatment period

### 1) Treatment period

The test drug treatment period begun at the first dose of the test drug on day 1 of cycle 1, and was consist of consecutive 28-day cycles of the test drug (QD or other regimens determined by the safety monitoring committee) administered orally in a capsule form. Here, one cycle consists of 28 days. Subjects participating in the clinical trial may continue to receive the administration of the test drug until the disease progresses and dose interruptions of the test drug follows the clinical trial guidelines and the investigator's judgment.

### 2) How to escalate dose of test drug

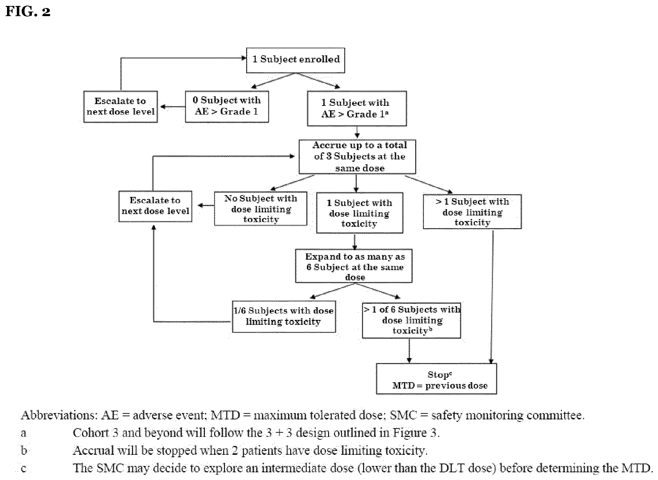

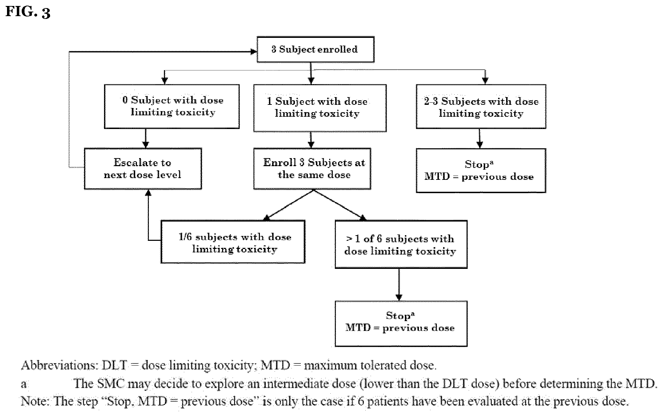

A method for escalating a dose during the treatment period (phase Ia part) is as follows. At least of 1 and up to 6 subjects may be enrolled in cohorts 1 and 2.3 to 6 subjects may be enrolled into subsequent cohorts. The first clinical trial subject in each cohort is treated and observed for 7 days before the test drug is administered to the next 2 clinical trial subjects in that cohort.
(1) If the first enrolled subject in cohort 1 experiences a Grade 2 or higher adverse event (AE) related to the test drug, additional subjects are enrolled at that cohort and all subsequent cohorts have a planned minimum of 3 subjects. If cohort 1 is successfully completed with only 1 subject, dose escalation will occur, and cohort 2 may start with 1 subject. Then if this first enrolled subject in cohort 2 experiences a Grade 2 or higher AE related to the test drug, additional subjects will be enrolled at that cohort and all subsequent cohorts have a planned minimum of 3 subjects (FIG. 2).
(2) Cohort 3 and all subsequent cohorts will follow the standard 3 + 3 design. For cohorts enrolling 3 subjects, if there are no dose limiting toxicity (DLT) in the first 3 subjects, dose escalation to the next higher dose level may proceed upon recommendation by the safety monitoring committee (SMC). If 1 of the 3 subjects experiences a DLT, that dose level will be expanded to 6 subjects (3 additional subjects will be enrolled). If 2 subjects at a given dose level experience a DLT, the maximum tolerated dose (MTD) will have been exceeded and further accrual to the cohort will be terminated. The next lower dose level will be expanded to 6 subjects or if 6 subjects have already been enrolled and no more than 1 of the 6 experienced a DLT, then this lower dose level will be considered the MTD (FIG. 3).
(3) A subject experiencing a dose limiting toxicity (DLT) may continue treatment with a reduced dose of the test drug once the DLT is resolved or if the DLT decreases to starting grade or Grade 1 (whichever is greater) prior to initiating the reduced dose. Should a dose-reduced subject experience another DLT, the subject should not receive further doses.
(4) Premature drop-outs are those subjects discontinued in cycle 1 who had < 20 of the 28 scheduled days of dosing, as follows:
   ① subjects who discontinued due to causes nominally unrelated to adverse events or unrelated to toxicity;
   ② subjects who discontinued due to causes other than adverse events or toxicity
(5) Premature drop-outs trigger addition of new subject in any cohort. This decision is to be made after discussion by the clinical trial team, sponsor and investigator and the addition will follow as below.
   ① If the subject is in a cohort planned for a single subject (cohorts 1 or 2), the premature drop-out will trigger the single-subject cohort to proceed to standard 3+3 design. Hence, the cohort will be expanded to a planned minimum of 4 subjects. Subsequent cohorts will be planned to have a minimum of 3 subjects following the standard 3+3 design. One DLT in this group will prompt expansion with an additional 3 subjects as in the standard 3+3 description above.
   ② If the premature drop-out subject is part of a cohort planned for 3 subjects, the cohort will be expanded to 4 subjects. One DLT in this group will prompt expansion with an additional 3 subjects as in standard 3+3 design.
   ③ If a subject drops out prematurely and the SMC does not recommend the adding of an additional subject to the cohort, the dropped-out subject will be treated as having experienced a DLT for this dose escalation decision process.
(6) The above dose limiting toxicity (DLT) corresponds to abnormal laboratory value of National Cancer Institute Common Terminology Criteria for Adverse Events (CTCAE) Grade ≥ 3 which the investigator has assessed as unrelated to disease, disease progression, intercurrent illness, or concomitant medications, which occurs within the first cycle of treatment with the test drug during the Phase Ia (dose escalation) part of the clinical trial. The dose limiting toxicity (DLT) in this clinical trial may include the followings:
   ① Any grade 5 toxicity.
   ② Hematologic dose limiting toxicities.
      (i) Any ≥ Grade 4 hematologic toxicity except Grade 4 neutropenia lasting ≤ 5 days, lymphopenia, or leukopenia at any grade.
      (ii) ≥ Grade 3 febrile neutropenia (an ANC <1000/mm3 and a single temperature of > 38.3°C (101°F) or a sustained temperature of ≥ 38°C (100.4°F) for more than 1 hour).
      (iii) Grade 3 neutropenia with infection.
      (iv) Grade 3 thrombocytopenia with bleeding.
   ③ Non-hematologic dose limiting toxicities.
      (i) Any ≥ Grade 3 toxicity during DLT period, excluding:
         - Grade 3 diarrhea of < 3 days duration following optimal therapy.
         - Grade 3 nausea and vomiting of < 3 days duration with optimal therapy.
         - Grade 3 fatigue < 7 days duration following initiation of adequate supportive care.
         - Asymptomatic Grade 3 liver function test (alanine aminotransferase [ALT], aspartate aminotransferase [AST], or gamma-glutamyl transferase [GGT]) increase that resolves to ≤ Grade 1 in less than 7 days after medical management.
         - Any other laboratory values out of normal range that are unlikely related to study treatment, do not have any clinical correlation, and resolve to ≤ Grade 1 within 7 days with adequate medical management.
      (ii) Grade 3 or higher skin reactions including dermatitis exfoliative, rash erythematous, rash generalized, rash macular, rash papular, exfoliative rash and skin disorders.
      (iii) Grade 2 pneumonitis which does not resolve within seven days after medical management.
   ④ Any clinically significant or persistent toxicities that may also be considered DLTs following review by the safety monitoring committee (SMC).

### C. Follow-up period

The follow-up period consists of a safety follow-up visit at 30±2 days after administration of the last dose of the test drug, a subsequent follow-up visit (if necessary), and an end-of-study (EOS) visit. Subjects who discontinue treatment should be followed up until resolution of toxicity or until confirmed disease progression.

### 4. Assessment method

### A. Test procedure and assessment

The trial assessment, procedure, and the schedule of activities are as summarized in table 4, and at scheduled time points, where multiple assessments are simultaneously scheduled, the order of assessments is as follows: electrocardiogram (ECG), vital signs, then blood sampling (PK sampling followed by PD sampling and safety laboratory sampling).

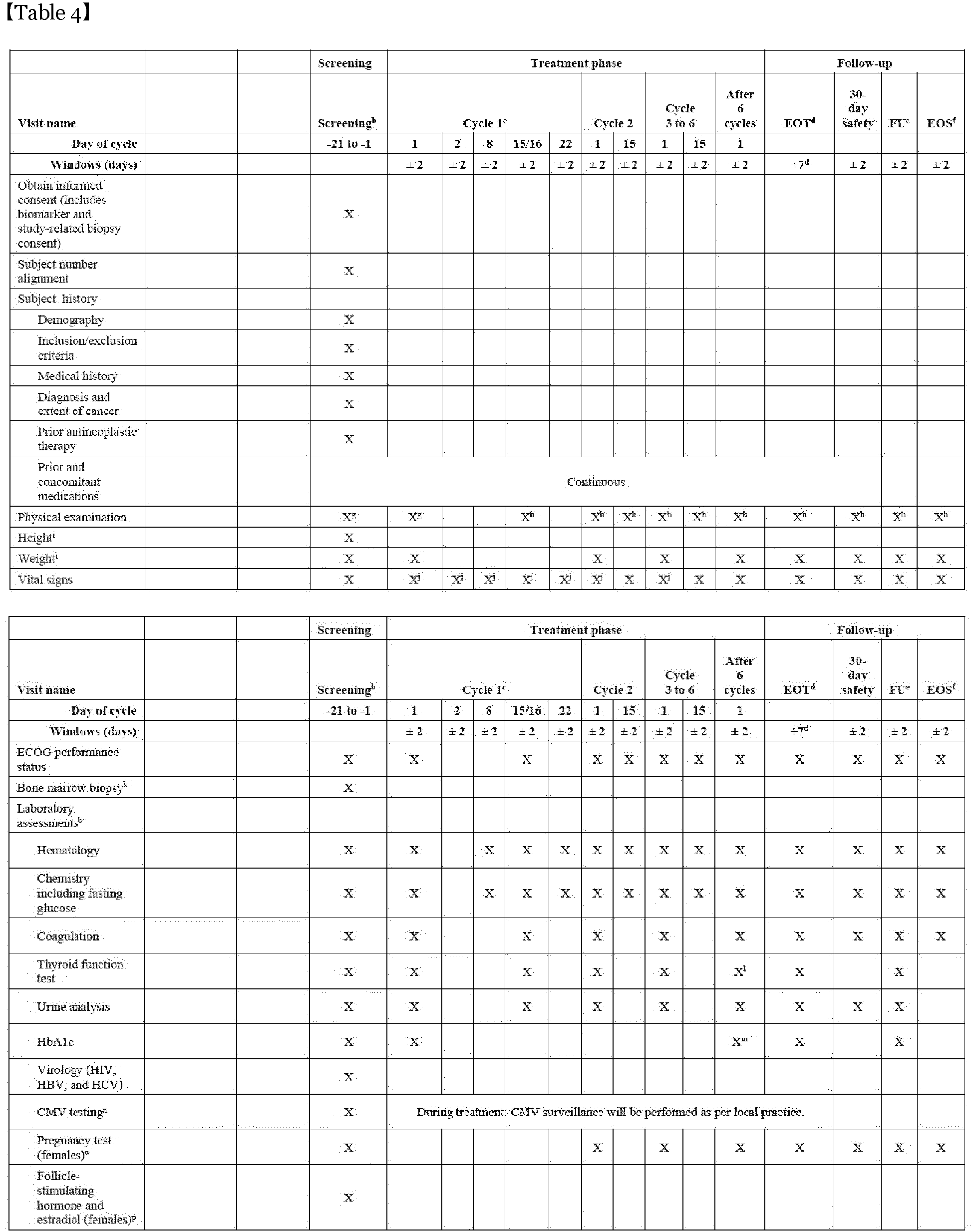

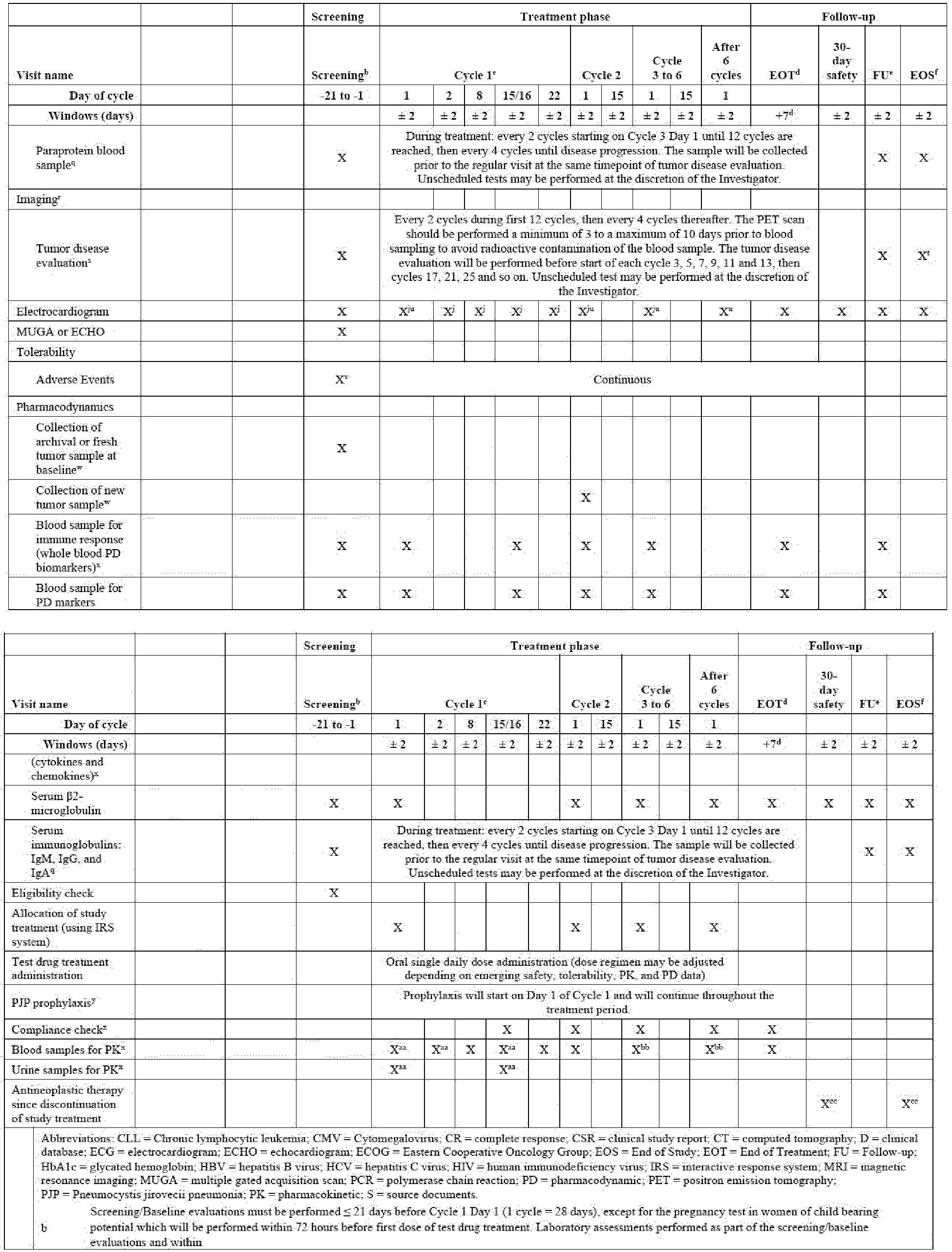

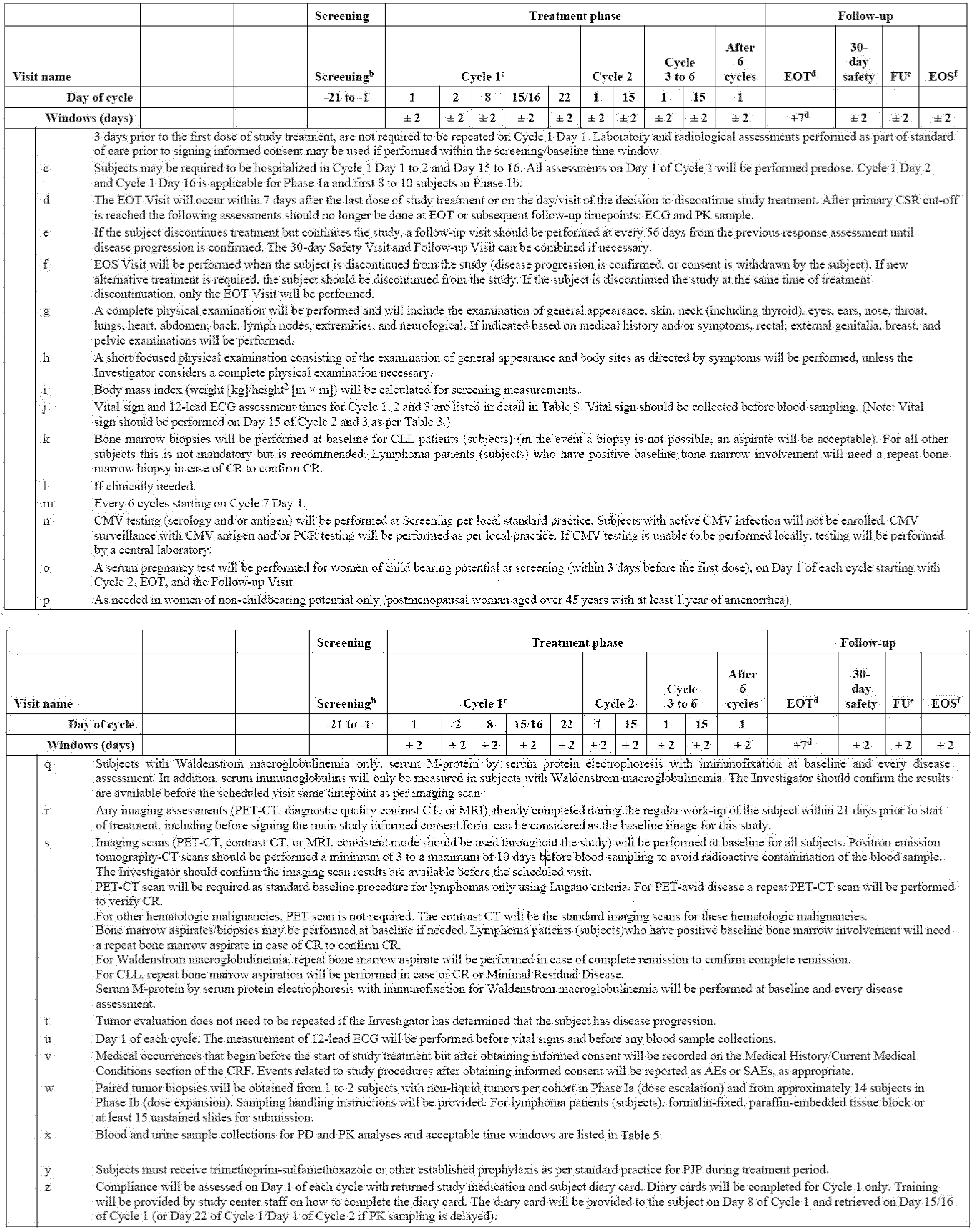

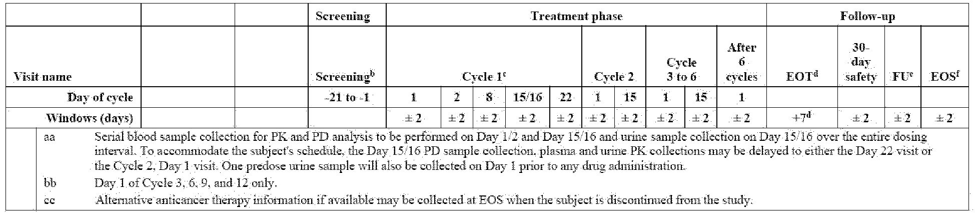

### B. Primary endpoints ... Assessment of safety and tolerability

1) Assessed by adverse events, vital signs, electrocardiogram (ECG), clinical laboratory tests, physical examination, ECOG performance status up to the last Safety Follow-up Visit, and baseline medical conditions.
2) Tolerability assessment includes review of dose interruptions, reductions, and doses administered up to the last Safety Follow-up Visit.
3) Determination of the maximum tolerated dose (MTD) and the starting dose (RP2D) for clinical phase Ib/II are based on dose limiting toxicity (DLT) during Cycle 1.

### C. Secondary endpoints ... Plasma and urine PK characterization and preliminary antitumor activity assessment

1) Plasma concentration-time profile of the test drug (days 1 and 15), urine concentration data (day 15), and derived single dose plasma PK and multiple dose plasma and urine PK parameters (if applicable).
   - Blood samples are collected according to a predetermined schedule to measure a plasma concentration of the test drug (Table 5). In phase Ia clinical trials, entire urine needs to be collected over each dosing interval according to a pre-determined schedule, starting after dose administration (Table 5). 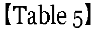 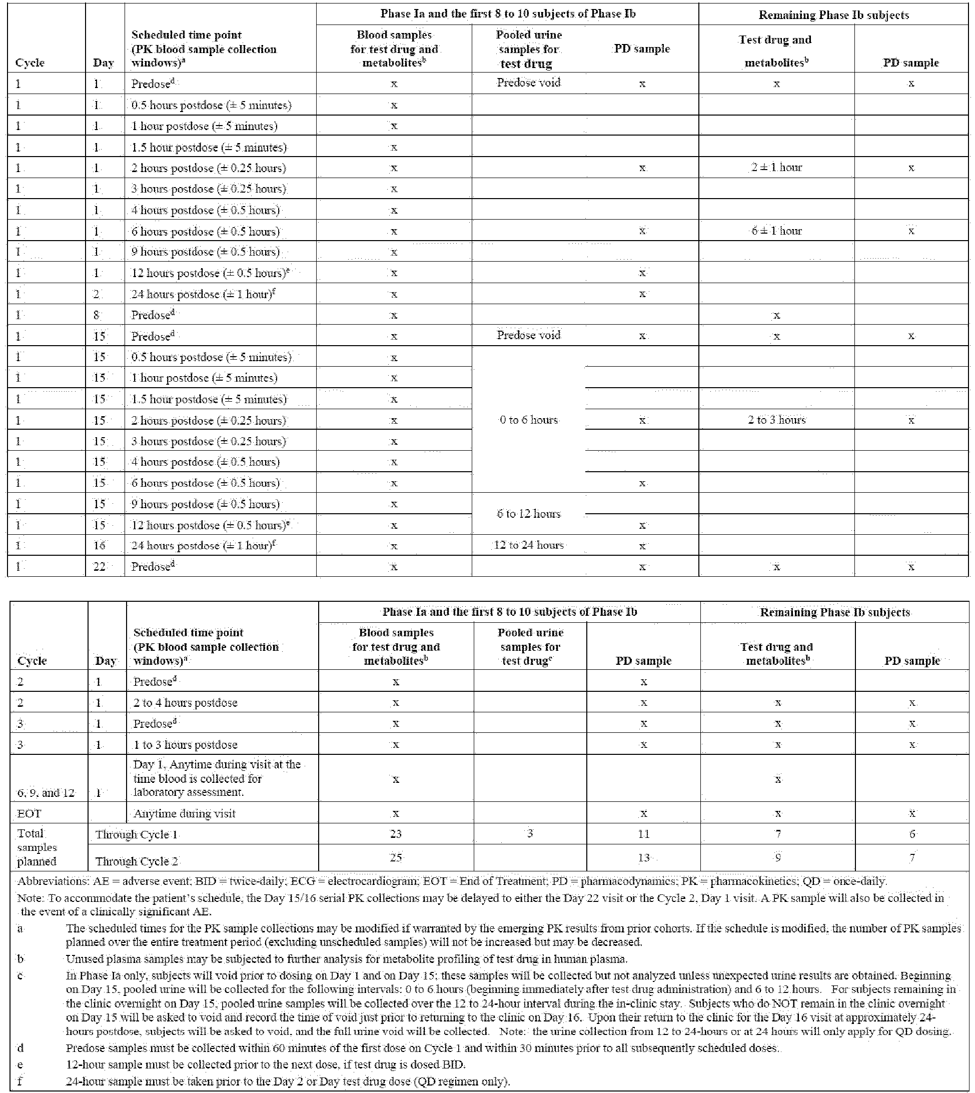
   - Samples for the determination of the test drug in plasma and urine are analyzed using appropriate validated bioanalytical methods.
   - Pharmacokinetic parameters are derived using noncompartmental methods with Phoenix^{®} WinNonlin^{®} Version 8.0 or higher (Certara, L.P. Princeton, New Jersey, USA) and/or SAS^{®} Version 9.2 or higher (SAS Institute, Inc., Cary, North Carolina, USA). Actual elapsed time from dosing will be used

for the final plasma PK parameter calculations. A minimum of 3 quantifiable postdose concentrations will be required for all calculations.
- The PK parameters to be determined for plasma concentration of the test drug on day 1 of cycle 1 and day 15 of cycle 1, if calculable, include but not be limited to the following parameters presented table 6. 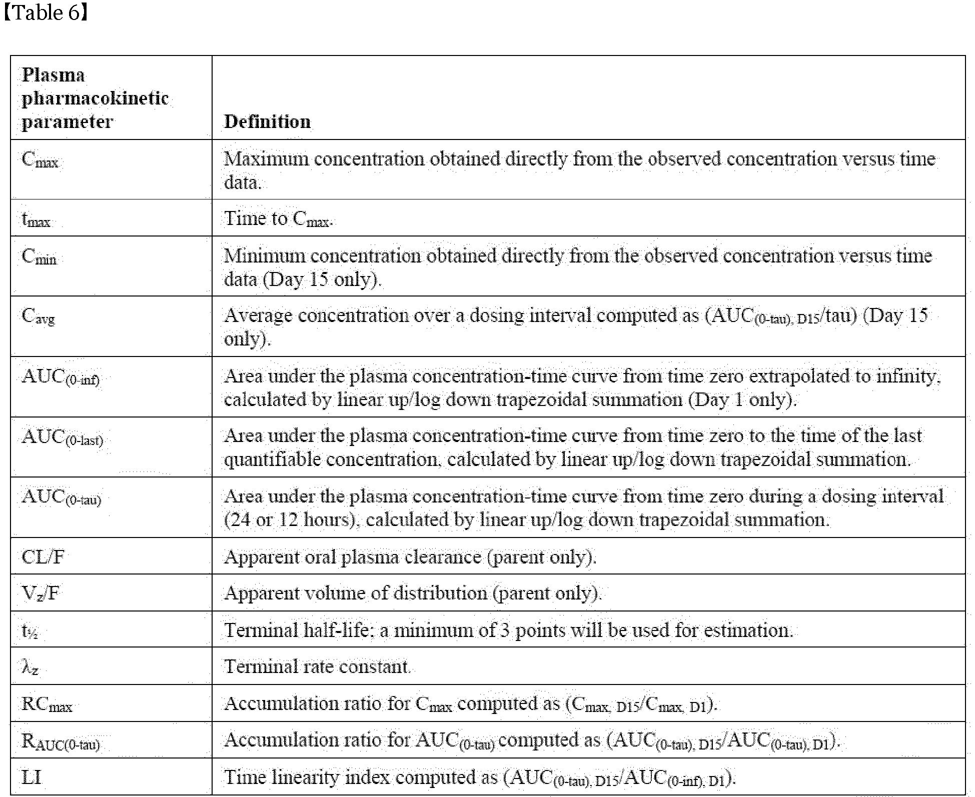
- The PK parameters to be determined for urine test drug concentration on cycle 1 day 15, if calculable, include but not be limited to the following parameters presented in Table 7.

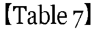

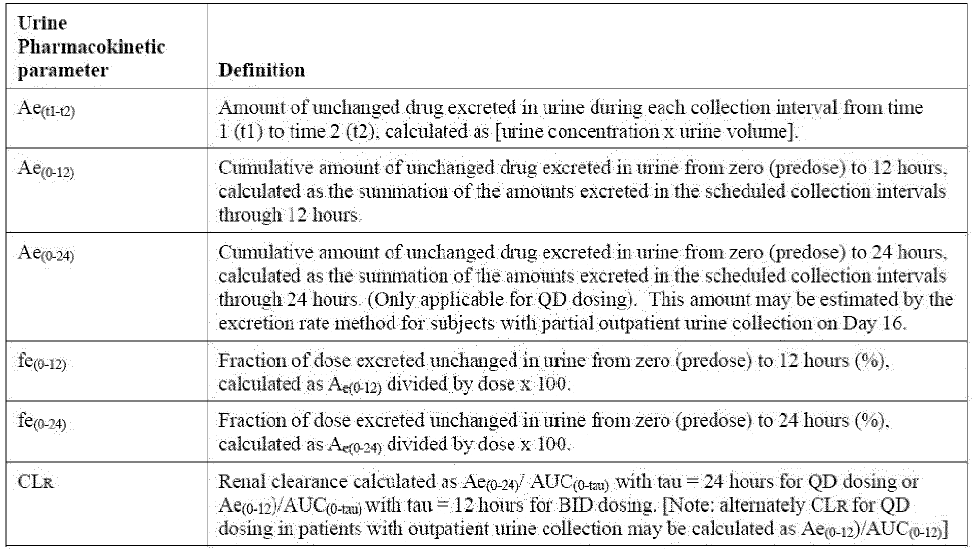

### D. Exploratory endpoints ... Pharmacodynamic (PD) characterization and biomarker investigation

1) Absolute and change from baseline in systemic cytokines and chemokines; phenotypic characterization of activated immune cells; changes from baseline in tumor biopsy assessment; and other pharmacodynamic (PD) biomarkers test drug exposure.
   - Exploratory pharmacodynamic (PD) and biomarker (immunogenicity) data may include analysis of the following:
      (i) Systemic cytokines and chemokines (serum): CCL3, CCL4, INF-γ, interleukin (IL)-6, IL-10, tumor necrosis factor (TNF)-α.
      (ii) Whole blood pharmacodynamic (PD) biomarkers:
         ① Regulatory T cells - programmed cell death protein 1 (PD 1), cytotoxic T cell lymphocyte-associated protein (CTLA)-4, lymphocyte activation gene (LAG)-3.
         ② Cluster of differentiation (CD)8 - PD 1, CTLA-4, LAG-3, T cell immunoglobulin (Ig) and mucin domain containing-3, T-cell immunoreceptor with Ig and immunoreceptor tyrosine-based inhibition motif domains, Ki67.
         ③ Myeloid-derived suppressor cells.
      (iii) Tumor biopsy: C-MYC (IHC, FISH), rH2AX, pAKT (T30S, S473)
   - Paired tumor biopsy is obtained from 1 to 2 subject(s) with non-liquid tumors per cohort in Phase Ia (dose escalation) and in Phase Ib (dose expansion) according to the planned schedule of activities to explore PD biomarkers. Similar information may be obtained from the standard tumor assessment for liquid tumors. For lymphoma subjects, a formalin-fixed, paraffin-embedded (FFPE) tissue block or at least 15 unstained slides for submission. The investigations that are chosen will depend on the availability of sufficient and suitable biological specimens and will be defined in the laboratory manual.
   - Exploratory biomarker analysis may be conducted on any remaining tumor and/or blood samples (including PK samples). In this trial, the search for other potentially relevant biomarkers to investigate the effect of the test drug as well as to determine how changes in biomarkers may relate to test drug exposure and clinical outcomes. These additional investigations are dependent upon dependent upon clinical outcome, reagent, and sample availability.
2) Profiling of test drug metabolites

### E. Safety assessments

### 1) Safety assessments items

Planned time points for all safety assessments are provided in table 4, and the methods are as follows.

### (1) Demographic and medical history

Demographic information and relevant medical and surgical history are recorded in an electronic case report form (eCRF).

### (2) Physical examinations

At screening and cycle 1 day 1, prior to administration of the test drug, complete physical examinations are performed, and are include the examination of general appearance, skin, neck (including thyroid), eyes, ears, nose, throat, lungs, heart, abdomen, back, lymph nodes, extremities, and neurological. If indicated based on medical history and/or symptoms, rectal, external genitalia, breast, and pelvic examinations are performed. At subsequent assessments short physical examinations consisting of the examination of general appearance and body sites as directed by symptoms are performed.

### (3) Vital signs

Vital signs (body temperature, pulse rate, respiratory rate, and blood pressure) are measured in the supine or sitting position during the trial and should be preceded by at least 5 minutes of rest in the supine or sitting position. The position is documented in the eCRF. Vital signs are collected prior to any simultaneously scheduled blood collections (e.g., PK or clinical laboratory samples). Additional vital signs measurements may be performed at any time during the trial at the discretion of the investigator, if medically indicated.

### (4) Height and weight

Height (only at screening) and body weight are measured according to the study center's standard procedures. the body mass index (BMI: weight [kg]/height² [m × m]) is calculated for screening measurements.

### (5) Performance status

Eastern Cooperative Oncology Group performance status (ECOG PS) is assessed as below (Table 8).

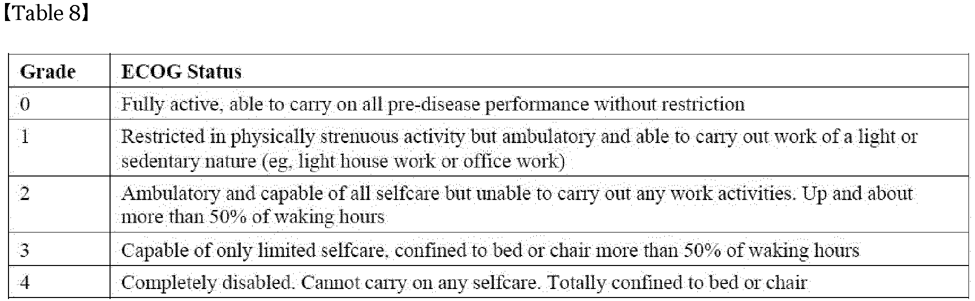

### (6) Electrocardiogram

Duplicate 12-lead electrocardiogram is obtained at times listed in the Schedule of Activities (Table 4) and the times listed in table 9, respectively, after resting for at least 5 minutes using an ECG machine that automatically calculates the heart rate and measures PR, QRS, QT, and QTc intervals. And the following parameters are recorded: Heart rate, PR interval, QRS duration, QT and QTcF.

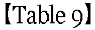

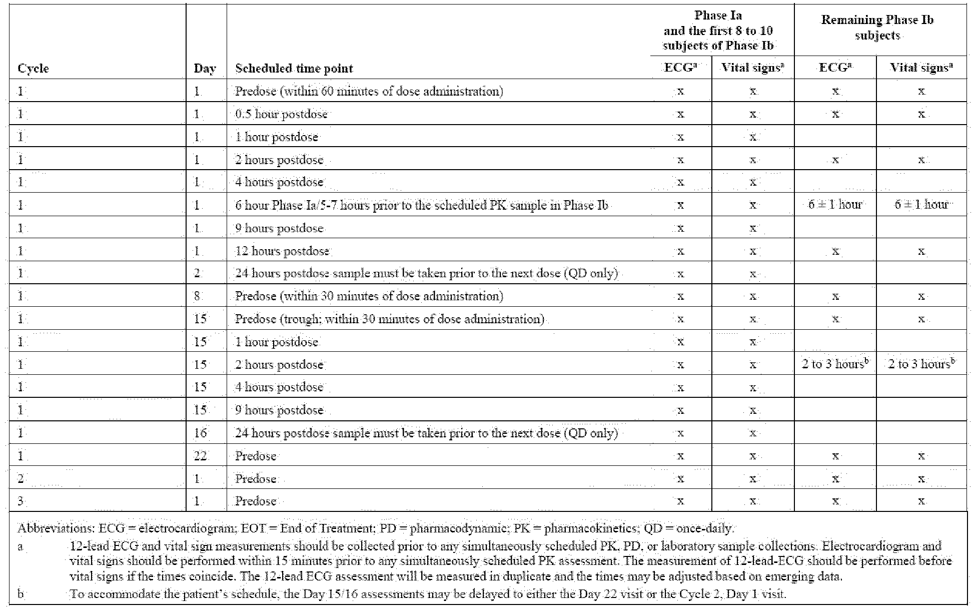

At each time point, 2 individual ECG tracings should be obtained as closely as possible in succession, but no more than 2 minutes apart. The duplicates should be completed in less than 5 minutes. After the primary CSR cut-off date is reached, it is not necessary to measure electrocardiogram (ECG) with the EOT assessments. Additional ECGs may be conducted for safety reasons at the discretion of the investigator.

### (7) Cardiac imaging

The left ventricular heart function is evaluated by ECHO or MUGA scan at screening/baseline. Additional cardiac imaging may be performed if indicated by clinical signs or symptoms. The same imaging modality should be used for the additional imaging.

### (8) Clinical safety laboratory assessments

A list of clinical laboratory tests to be performed is presented below (Table 10). See table 4 for inspection timing and frequency.

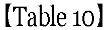

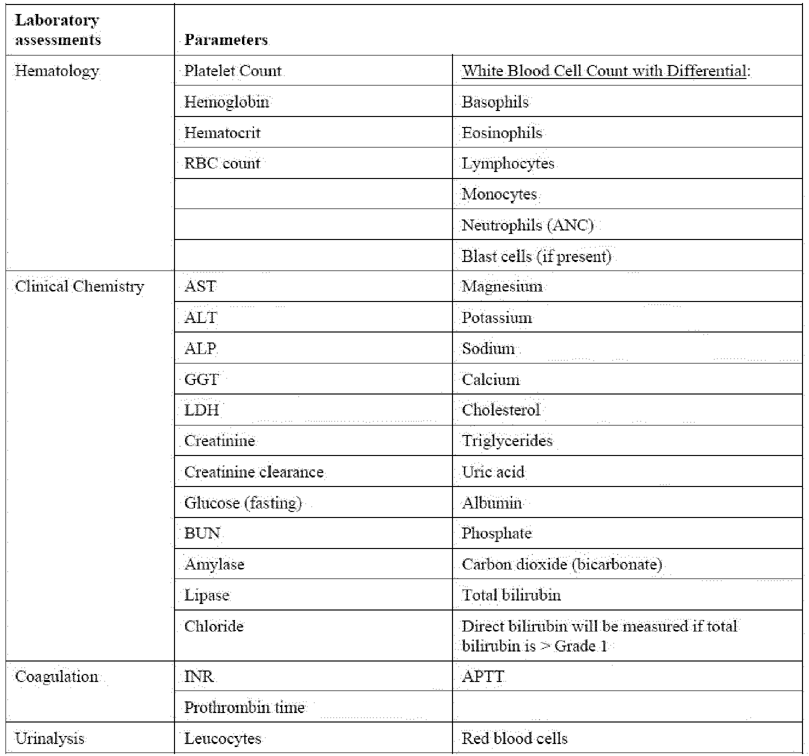

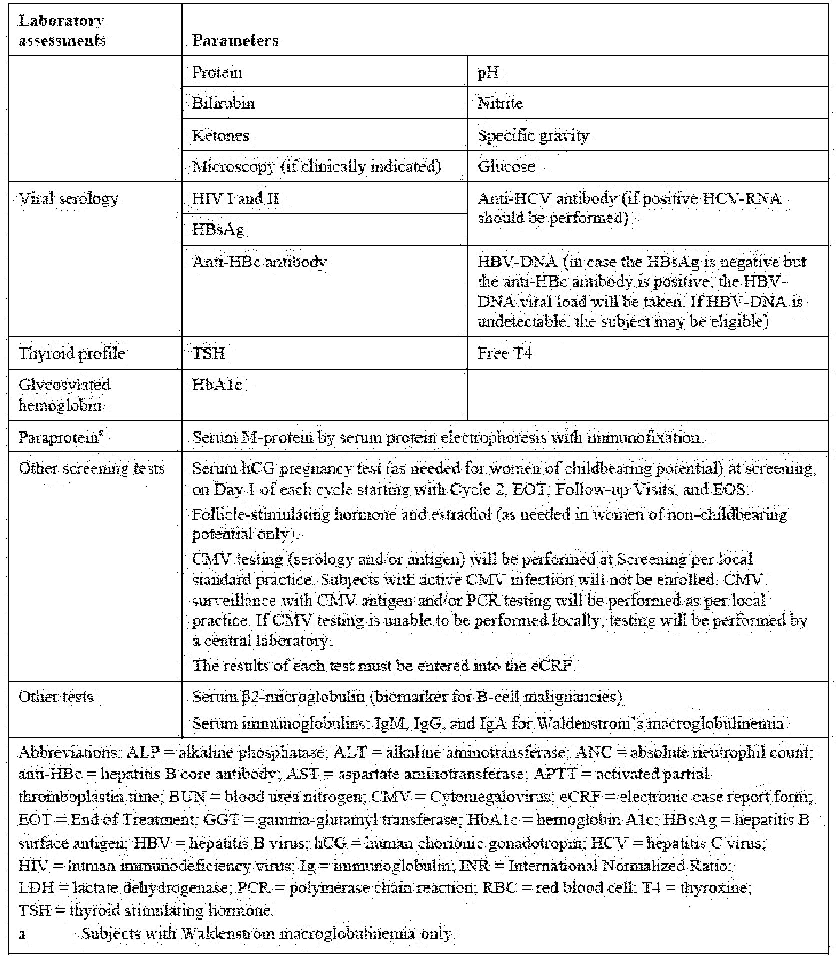

All laboratory tests with values considered clinically significantly abnormal during participation in the clinical trial or within 30 days after the last dose of test drug treatment should be repeated until the values return to normal or baseline or are no longer considered clinically significant by the investigator or medical monitor. On the day of administration of the test drug, samples for these parameters are collected prior to dose administration. Additional evaluations may be performed at the investigator's discretion if medically indicated. In addition, laboratory assessments may be performed if a subject has provided a paired biopsy tumor sample for analysis of biomarkers.

At screening, a serum pregnancy test must be performed within 3 days before the first dose. During the study (on Day 1 of each cycle starting with Cycle 2), EOT, Follow-up Visits, and EOS a serum pregnancy test must be performed. If a test subject (female) becomes pregnant, administration of the test drug needs to be stopped immediately. If a pregnancy test is positive, but the subject is thought not to be pregnant, the test drug administration should be delayed until it is determined that the test was falsely positive and pregnancy is excluded.

### 2) Adverse events

### (1) Definition of adverse event and serious adverse event

An adverse event (AE), as defined in this clinical trial, is any untoward medical occurrence in a subject, temporally associated with the use of test therapy, whether or not considered related to the test therapy (Table 11).

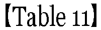

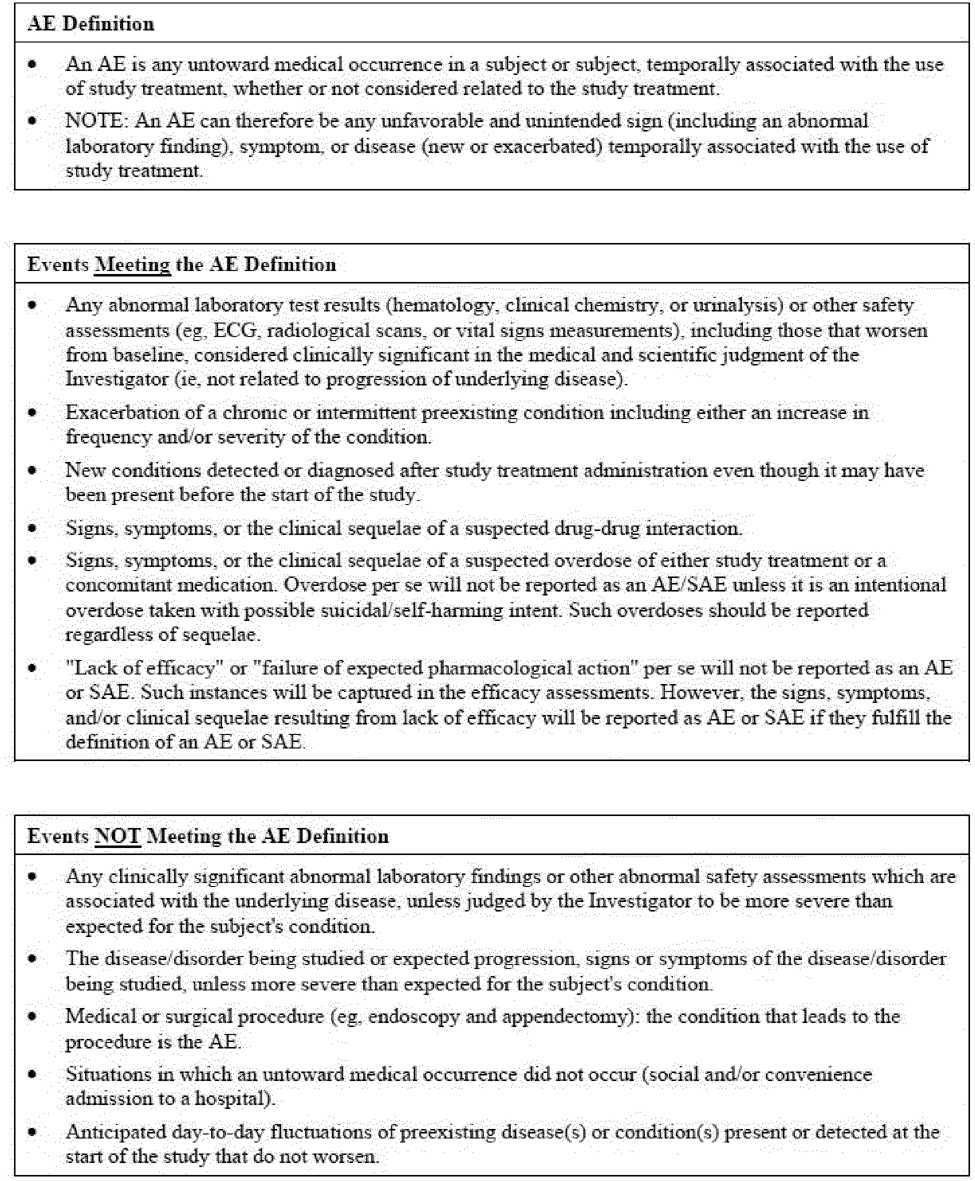

In this clinical trial, if an event does not correspond to the above-defined adverse events, then it cannot be a serious adverse event (SAE) even if serious conditions are met (e.g., hospitalization for signs/symptoms of the disease under clinical trial or death due to progression of disease). The definition of serious adverse event is summarized below (Table 12).

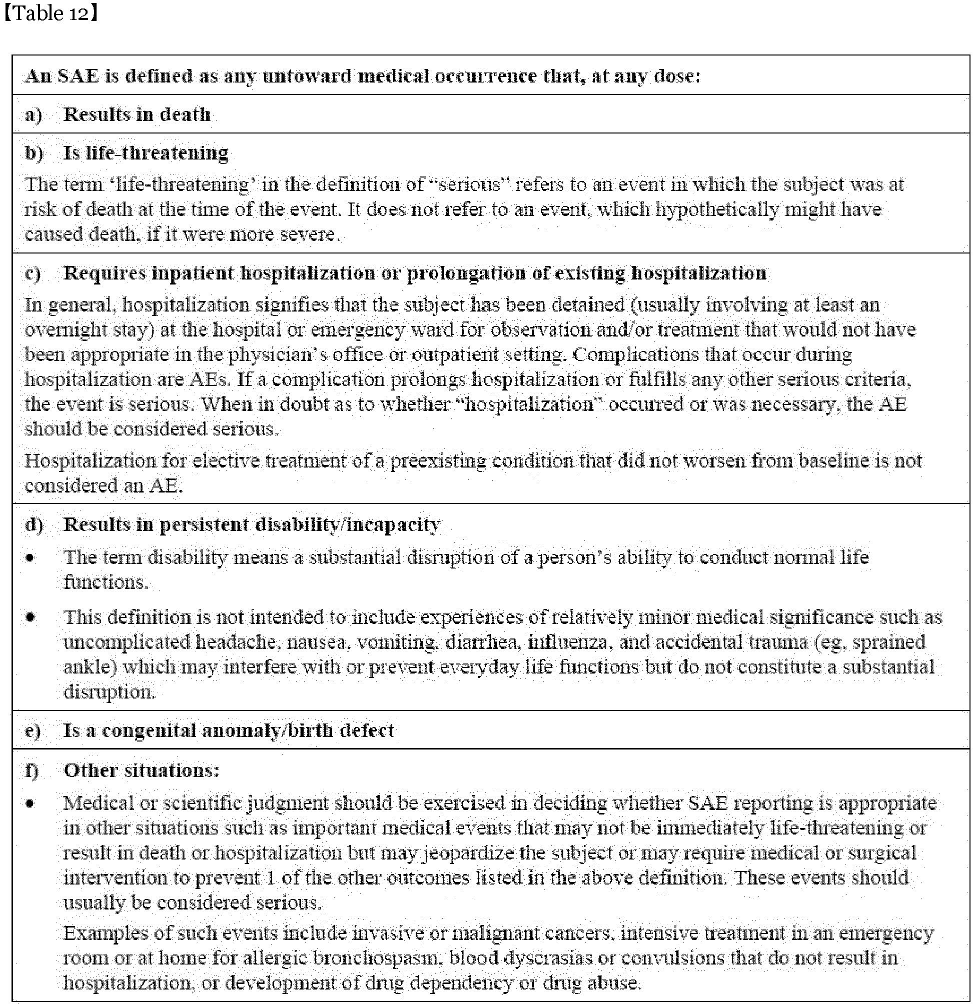

In addition, the severity assessment criteria of this clinical trial are summarized below (Table 13).

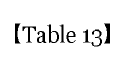

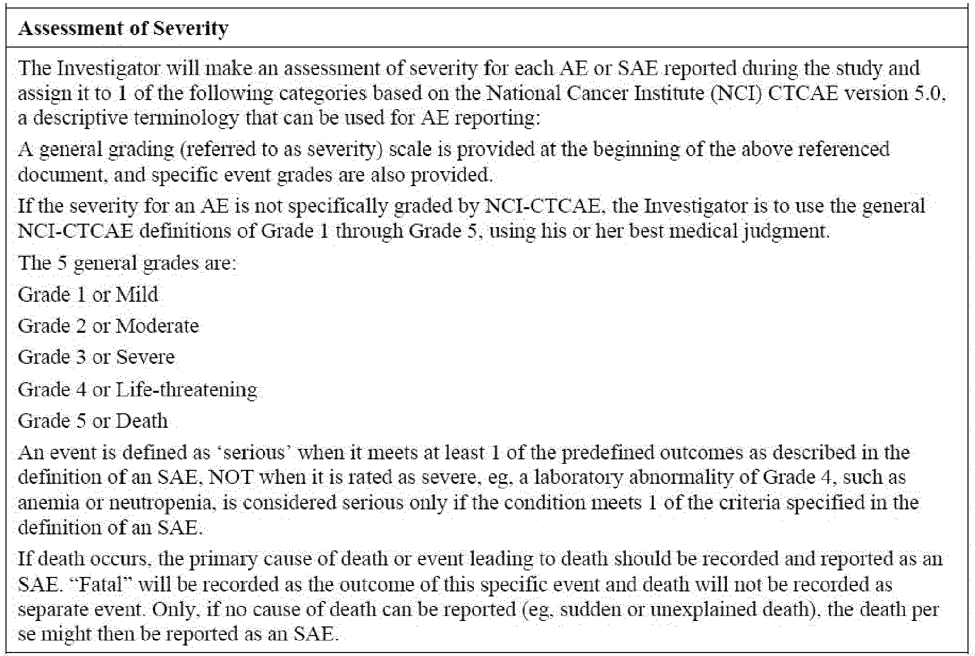

- All adverse events (AEs) and serious adverse events (SAEs) are collected from the signing of the informed consent form (ICF) until the 30-day Safety Follow-up at the time points specified in the schedule of activities.
- Care needs to be taken not to introduce bias when detecting AEs and/or SAEs. And Openended and non-leading verbal questioning of the subjects is the preferred method to inquire about AE occurrences.
- Details of all pregnancies in female subjects and female partners of male subjects are collected after the start of test drug administration and until 3 months after the last administration of test drug. Abnormal pregnancy outcomes (e.g., spontaneous abortion, fetal death, stillbirth, congenital anomalies, and ectopic pregnancy) are considered SAEs.

### F. Efficacy assessment

1) Tumor response is assessed for both phases Ia and Ib. The anticancer activity of the test drug is assessed by applying various criteria through imaging tests every two cycles of clinical trials and at the end of therapy (EOT) (optional) (see table 14 for disease assessment criteria below), or for liquid tumors, bone marrow aspirates/biopsies and blood assessments are performed. Tumor response parameters that are assessed include the following:
   (1) Best overall response: complete response (CR), partial response (PR), stable disease (SD, lasting 12 weeks), or disease progression (DP)
   (2) Objective response rate (ORR): complete response (CR) + partial response (PR)
   (3) Clinical benefit rate (CBR): complete response (CR) or partial response (PR) or stable disease (SD) lasting at least 12 weeks
   (4) Duration of response or duration of SD
   (5) Time-to-event endpoints of time to progression (TTP), progression-free survival (PFS), and overall survival (OS) during the test observation period are also assessed.

### 2) Disease assessment criteria

Details of the disease assessment criteria and the tools used to determine efficacy parameters are as follows (Table 14).

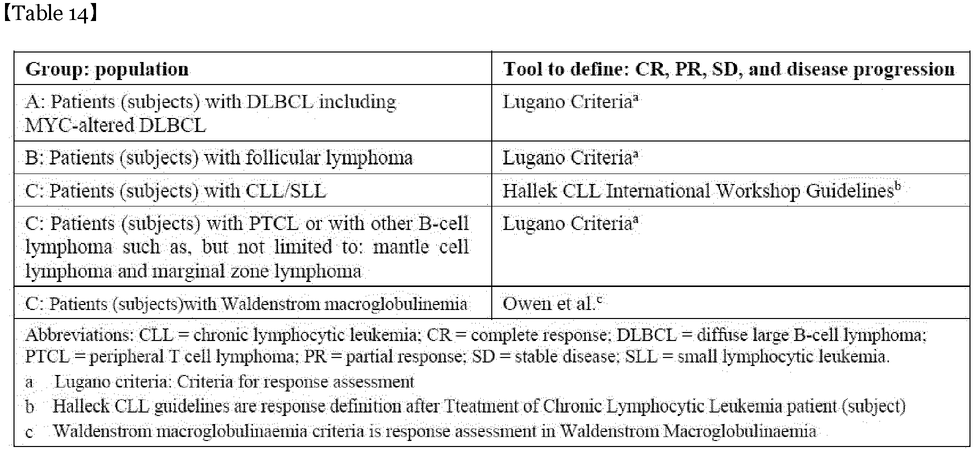

### 3) Baseline tumor assessments

At screening, potential subjects with lymphoma undergo a positron emission tomography (PET) scan with 2 deoxy-2-[fluoro-18]fluoro-D-glucose along with a PET-CT scan. The scan should be performed a minimum of 3 to a maximum of 10 days prior to blood sampling to avoid radioactive contamination of the blood sample.

At screening, a diagnostic quality CT scan with IV contrast of chest/abdomen/pelvis/additional known lesions is performed for all subjects (for those subjects who are imaged with PET-CT, the imaging studies may be combined). If at screening, a subject has a medical contraindication to the CT IV contrast or develops a contraindication during the clinical trial, the following radiologic assessments are performed: a non-contrast chest CT and a contrast abdomen/pelvis magnetic resonance imaging (MRI). Chest x-rays and ultrasound should not be used to measure tumor lesions.

If available and of acceptable quality (e.g., CT portion of PET/CT scan was performed with contrast), a previously performed PET/CT scans that were done within 21 days prior to start of treatment may be used for baseline radiology assessment.

All subsequent disease assessments are performed with CT scan with IV contrast. For subjects staged with CT, up to 6 of the largest target nodes, nodal masses, or other lymphomatous lesions that are measurable in 2 diameters (longest diameter [LDi] and shortest diameter) should be identified from different body regions representative of the subject's overall disease burden and include mediastinal and retroperitoneal disease, if involved. A measurable node must have an LDi greater than 1.5 cm. Measurable extranodal disease may be included in the 6 representative, target lesions. A measurable extranodal lesion should have an LDi greater than 1.0 cm. All other lesions (including nodal, extranodal, and assessable disease) should be followed as non-target disease.

Brain MRI or CT needs to be completed at screening only if clinically indicated. Contrast-enhanced brain MRI is preferred. However, if MRI contrast is contraindicated, then MRI without contrast or CT with/without contrast is acceptable.

### 4) Baseline tumor assessment

Each lesion that is measured at baseline must be remeasured by the same method (either radiologic or nuclear method) throughout the clinical trial so that the comparison is consistent. For CT scans there is a ± 7 days window for the scheduled assessment. Subjects with PET-avid disease will have a repeat PET-CT to verify complete response (CR). The PET-CT scans should be performed a minimum of 3 to a maximum of 10 days prior to blood sampling to avoid radioactive contamination of the blood sample. Additional imaging assessments may be performed at any time during the clinical trial at the investigator's discretion to support the efficacy evaluations for a subject, as necessary. Clinical suspicion of disease progression at any time requires a physical examination and imaging assessments to be performed promptly rather than waiting for the next scheduled imaging assessment. If an off-schedule imaging assessment is performed because progression is suspected, subsequent imaging assessments should be performed in accordance with the original imaging schedule.

For CR, after consultation with the medical monitor, a CR confirmatory PET or PET-CT may be performed. The PET-CT scan should be performed a minimum of 3 to a maximum of 10 days prior to blood sampling. In lymphoma subjects with bone marrow involvement prior to the clinical trial start a bone marrow biopsy should be performed to confirm CR.

### G. Population for analysis

The definition of an analysis group is as follows (Table 15).

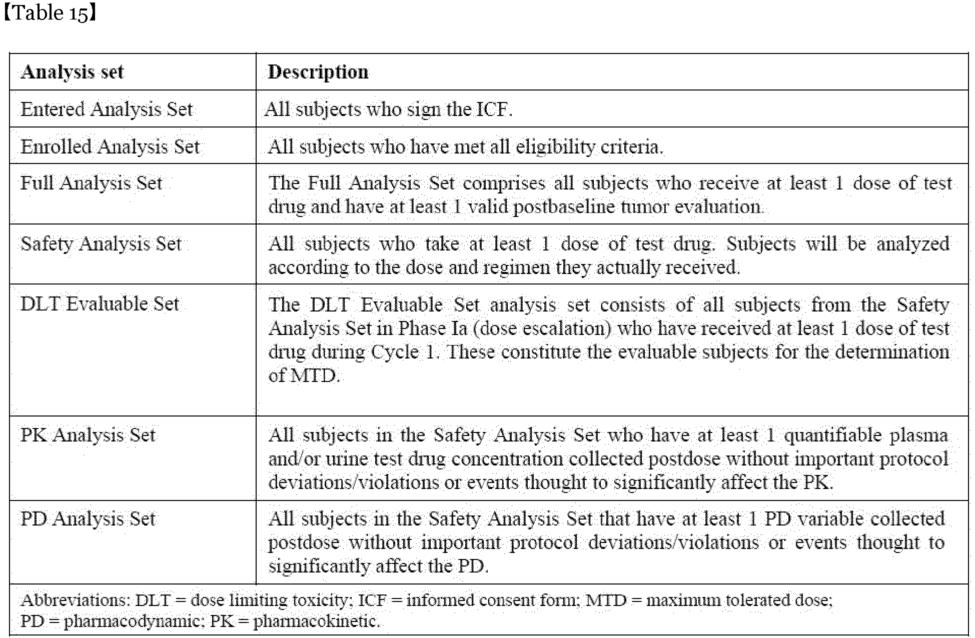

### 5. Statistical method

### A. Efficiency analysis

The tumor response (lymphoma (B-cell and T-cell), CLL/SLL, Waldenstrom macroglobulinemia) following administration of the test drug in phases Ia and Ib is assessed using the criteria in table 14. Tumor response parameters vary according to the above applied criteria and may include the followings:

### 1) Best overall response: Complete response (CR), partial response (PR), stable disease (SD) or disease progression (DP)

The best overall response is defined as the best response across all time points (in the order of CR, PR, SD, and DP) post-treatment. The best overall response (CR, PR, SD, or DP) is summarized by number of subjects and percentages. In the case of best response equal to SD, the minimum duration for SD should be met. For the purpose of calculating an SD rate, the rate will be the percentage (%) of subjects who continue to have SD for at least 12 weeks.

### 2) Objective response rate (ORR): CR + PR

The objective response rate (ORR) is the proportion of subjects with a best overall response of complete response (CR) or partial response (PR). The ORR and its 95% confidence interval (CI) are presented. The 95% (exact) CI is calculated based on the Clopper-Pearson method.

### 3) Clinical benefit rate (CBR): CR or PR or SD lasting at least 12 weeks

Clinical benefit rate (CBR) is defined as the proportion of subjects who achieve complete response (CR), partial response (PR), or durable stable disease (SD) (SD ≥ 12 weeks) based on the Lugano revised criteria for response assessment. The CBR as well as 95% (exact) CI are estimated by the Clopper-Pearson method.

### 4) Duration of response

Duration of response for responders (CR or PR) is defined as the time interval between the date of the earliest qualifying response and the date of disease progression or death for any cause, whichever occurs earlier. For subjects who are alive without disease progression following the qualifying response, duration of response is censored on the date of last evaluable tumor assessment or last Follow-up Visit for disease progression. Duration of response is summarized in subjects with a complete response (CR) or partial response (PR) using the Kaplan-Meier method. Median event time, quartiles Q1, Q3 and 2-sided 95% CI for the median is provided using the Brookmeyer-Crowley method.

### 5) Duration of SD

Duration of stable disease (SD) is defined as the time interval, in the absence of either complete response (CR) or partial response (PR) between the date of the first-time achieving SD after administration of test drug and the date of disease progression or death for any cause, whichever occurs earlier. For subjects who are alive without disease progression, duration of SD is censored on the date of last evaluable tumor assessment. Duration is summarized in subjects with a SD using the Kaplan-Meier method. The median event time, Q1, Q3, and 2-sided 95% CI for the median is provided using the Brookmeyer-Crowley method.

### 6) Time to tumor progression (TTP)

Time to tumor progression (TTP) is defined as the time from the date of first study dose to disease progression. Subjects without disease progression are censored at the date of 'last evaluable tumor assessment' or date of first dose, whichever comes later. If no postbaseline assessment is available, TTP is to be censored at the date of first dose. Time to progression is presented graphically as Kaplan-Meier curves. The median event time, Q1, Q3 and 2-sided 95% CI for the median is provided using the Brookmeyer-Crowley method.

### 7) Progression-free survival

Progression-free survival (PFS) is defined as the time from the date of first study dose to disease progression or death whichever occurs first in subjects. Subjects without event (no disease progression or death) are censored at the date of 'last evaluable tumor assessment'. Subjects for whom no postbaseline evaluable tumor assessments are available are censored at the time of first dose. Progression-free survival is summarized in the same way as tumor progression (TTP).

### 8) Overall survival during study observation period

Overall survival (OS) is defined as the time from the date of first dose until the date of death from any cause. For subject who is not known to have died by the EOS follow-up, OS is censored at the date of last contact. Date of last contact is defined as the latest of the following dates:
Treatment discontinuation date, last dosing administration date, last disease assessment date or the last follow-up date on which the subject was known to be alive. Overall survival will be presented graphically as Kaplan-Meier curves. Overall survival will be summarized in the same way as progression-free survival (PFS).

### B. Safety analysis

### 1) Phase Ia (dose escalation)

The number and proportion of subjects experiencing the dose limiting toxicity (DLT) are summarized based on the dose-limiting toxicity evaluable set. The corresponding 95% exact CI according to the Clopper-Pearson method is provided. Estimation of maximum tolerated dose (MTD)/recommended dose for clinical phase Ib/II (RP2D) are based upon the methods for determination of MTD/RP2D provided herein (see 3. Overall design, 2) How to escalate dose of test drug).

### 2) Phase Ia (dose escalation) and phase Ib (dose expansion)

The following analyses are performed on the safety analysis set.
(1) Treatment emergent adverse event (TEAE):
   A treatment emergent adverse event (TEAE) is defined as any AE that occurs after the first administration of the test drug through 30 days after the last administration of the test drug, or any event that is present at baseline but worsens in severity. The TEAE are to be coded and grouped using the Medical Dictionary for Regulatory Activities (MedDRA) Version 21.1 or higher. All the adverse events and toxicities are graded according to CTCAE (version 5.0). Analysis may include, but is not limited to, the followings:
   TEAEs, treatment-related TEAEs, Grade 3 or higher TEAEs, Grade 3 or higher treatment-related TEAEs, TEAEs resulting in discontinuation of test drug, serious TEAEs, serious treatment-related TEAEs, and TEAEs leading to death. The number and percent of subjects are summarized for the above items, and also summarized according to MedDRA System Organ Class (SOC) and Preferred Term (PT). A 2-sided 95% CI for the percentage of subjects in each TEAE category is provided. The severity and causality of the TEAE are summarized. All AEs occurring on the test are presented in data listing and the TEAEs are flagged.

Additional summaries are also provided using adverse events of special interest. The adverse events of special interest may include, but are not limited to, the followings:
Hepatotoxicity, skin rash, diarrhea/colitis, pneumonitis, decrease leukocytes, anemia, increased rates of infection including opportunistic infections such as PJP and CMV infection.
(2) Clinical laboratory parameters (hematology, clinical chemistry, coagulation, thyroid, glycosylated hemoglobin [HbA1c] and urinalysis) are summarized at each scheduled time. Changes over time are described using descriptive statistics, and the percentage of subjects with values outside the normal range is presented. In addition to the high and low laboratory assignments, clinically significant laboratory assessments are also identified. Incidence of abnormal values according to clinically significant laboratory evaluation results are provided. Clinical laboratory data is graded according to CTCAE. For laboratory tests where grades are not defined by CTCAE, results are graded by the low/normal/high classifications based on laboratory normal ranges. Frequency tables are used for newly occurring on-treatment Grades 3 or 4. Shift tables are produced for the comparison of baseline and postbaseline values at each sample time point for the selected laboratory parameters. Descriptive statistics are used to summarize vital sign results (pulse rate, respiration rate, body temperature, systolic and diastolic blood pressure) and changes from baseline. Data from the ECG central reviewer are used in summary presentations. The 2 values of each ECG parameter within a time point from the central reviewer are averaged to determine time specific parameter for a subject and used in summaries. Electrocardiogram variables are summarized using descriptive statistics at each scheduled time point, including change from baseline. Number and percentage of subjects with normal and abnormal results as assessed by the central reviewer for the overall interpretation are tabulated for each time point. Shift tables from baseline to worst postbaseline are produced.
(3) ECOG performance status is summarized by each visit.
(4) The physical examination is listed only. All clinically significant abnormal findings are recorded as medical history or adverse events.
(5) The results of pregnancy tests for females are listed.
(6) Number of cycles administered, administered dose, cumulative dose, dose intensity, relative dose intensity, and the overall duration of exposure are summarized for the Safety Analysis Set (SAS) and are summarized using standard summary statistics. The total dose per cycle for each subject is summarized using descriptive statistics. Tolerability of the test drug is assessed by summarizing the number of dose delays, interruptions, and dose reductions. Reasons for dose delays, interruptions, and dose reductions are listed by subject and summarized. The reasons for discontinuation from treatment are summarized and listed, along with dates of first and last administration of the test drug, the duration of exposure to the test drug, and the date of discontinuation of administration.
(7) All pre-medication and concomitant drug data are listed. Prior medications are those medications that are stopped prior to first dose of clinical trial treatment. Concomitant medications are medications taken at least once on or after first dose of clinical trial treatment. Medications started prior to and stopped on the same day as first dose of study treatment are considered as prior medication only. All medications are coded using the most recent version of the WHO Drug Dictionary/WHO Drug Reference List. The number and percentage of subjects is presented by PT and Anatomical Therapeutic Chemical Classification System level 1 and level 2 for prior and concomitant medications. All prior and concomitant medication data is listed.
(8) Non-medication therapy name is coded using MedDRA. The number and percentage of subjects is presented by PT. Non-medication therapy information is listed.
(9) Demographics, baseline medical status, medical history, and other baseline characteristics are summarized and listed for full analysis set (FAS).

### C. Pharmacokinetic (PK) analysis

Plasma concentrations and PK parameters of the test drug are listed and summarized by dose, cycle, and clinical trial phase using descriptive statistics.

Urine concentrations and parameters of the test drug are listed and summarized for each dose using descriptive statistics. The plasma concentration-time profile of the test drug is displayed graphically.

Scatter plots of individual values and geometric means of PK exposure parameters, as applicable, including area under the plasma concentration-time curve from time zero during a dosing interval [AUC₍₀₋ₜₐᵤ₎], area under the plasma concentration-time curve from time zero extrapolated to infinity [AUC(_{0-inf)}], or area under the plasma concentration-time curve from time zero to the time of the last quantifiable concentration [AUC₍₀₋ₗₐₛₜ₎] if AUC_{(0-inf)} is not calculable in a majority of subjects, and Cₘₐₓ versus dose will be presented by study day. Dose proportionality of PK exposure parameters Cₘₐₓ, AUC₍₀₋ₜₐᵤ₎, and AUC_{(0-inf)} [or AUC₍₀₋ₗₐₛₜ₎: if AUC_{(0-inf)} is not calculable in in most subjects] over the administered dose range are investigated using a power model. Time linearity and test drug accumulation may be evaluated by using an analysis of variance model. Effect of sex, age, and/or body weight may be explored graphically or by utilizing inferential analyses. Further details on any planned statistical analyses are provided in the Statistical Analysis Plan (SAP). Attainment of steady-state conditions are visually evaluated by graphical display of predose the test drug concentrations.

### D. Pharmacodynamic (PD) analysis

Exploratory PD and predictive biomarker data are listed and, if appropriate, presented in descriptive summaries for observed and change from baseline values.

### E. Pharmacokinetic (PK)-pharmacodynamic (PD) analysis

Relationship between test drug exposure and tumor size reduction may be explored graphically by means of scatter plots.

### F. Interim analysis

Details of the interim analyses to be conducted are described in a statistical analysis plan (SAP).
1) Phase Ia (dose escalation): Two interim analyses are done during the Phase Ia trial as follows.
   - After completion of cycle 1 of the last subject in cohort 3.
   - After completion of cycle 1 of the last cohort in phase 1a.
2) Phase Ib (dose expansion): One interim analysis is performed when all subjects in phase 1b have been treated or followed for enough time to have completed at least 6 cycles of treatment or have been followed to treatment discontinuation if less than 6 cycles.

### 6. Assessments results

### A. Safety assessment results

It was confirmed that 2 of 11 clinical trial subjects showed grade 3 skin reaction and 3 clinical trial subjects showed hepatotoxicity. All of the above adverse events were manageable and recovered to grade 0 or 1 upon discontinuation of the test drug.

The safety assessment results for 5 clinical trial subjects (DLBCL (n = 1) and PTCL (n = 4)) included in cohorts 1 to 3 are as follows:
Treatment-emergent adverse events (TEAEs) occurred in 4 out of 5 (80.0%) subjects. Of those events, the TEAEs in 3 subjects were evaluated to be related to the test drug (60.0%, 3 cases), and no administration-related deaths occurred. 2 subjects showed TEAE of grade 2 (moderate), and respectively 1 subject showed the TEAE of grade 1 (mild) and grade 4 (life-threatening). The grade 4 TEAE was thrombocytopenia and was not related to the test drug.

According to the above results, no serious adverse events rated as grades 3 (severe) to 5 (death) occurred in cohorts 1 to 3, and thus it was confirmed that there was no dose limiting toxicity (DLT) in the dose range of cohorts 1 to 3.

The safety assessment results for cohort 4 are as follows:
In cohort 4 (325 mg QD), two dose limiting toxicities (DLTs) were observed. Accordingly, it was considered that the maximum tolerated dose (MTD) was exceeded, and the expansion of the dosing to the cohort was terminated. The administered dose was de-escalated to the previous lower dose level (200 mg QD) and extended to enrollment of 3 additional subjects for the de-escalated dose. No dose limiting toxicity was confirmed in 6 subjects in the expansion cohort (200 mg QD).

From the safety assessment results for the above cohorts 1 to 4, it was confirmed that the maximum tolerated dose (MTD) was 200 mg QD, and it was confirmed that the tolerability was excellent in this dose range. In addition, 200 mg QD was recommended as the starting dose (RP2D) for clinical phase Ib/II by the Safety Monitoring Committee.

### B. Efficacy assessment results ... Tumor response assessment

Tumor response assessment was confirmed in 11 of 12 subjects enrolled in clinical phase Ia (dose escalation part). Specifically, a complete response (CR) in 1 subject and a partial response (PR) in 2 subjects were confirmed, and stable disease (SD) were observed in 6 subjects. In addition, disease progression (PD) was confirmed in 2 subjects.

Among 12 enrolled subjects, in particular, in the assessment of tumor response of 8 subjects diagnosed with peripheral T-cell lymphoma (PTCL), 1 subject with complete response (CR), 2 subject with partial responses (PR), 4 subject with stable diseases (SD) and 1 subject with disease progression (PD) were observed, and a disease control rate (DCR) was 87.5%.

From the above results, it was confirmed that the test drug showed preliminary activity in peripheral T-cell lymphoma (PTCL) subjects.

### C. Pharmacokinetic and pharmacodynamic assessment results

### 1) Pharmacokinetics

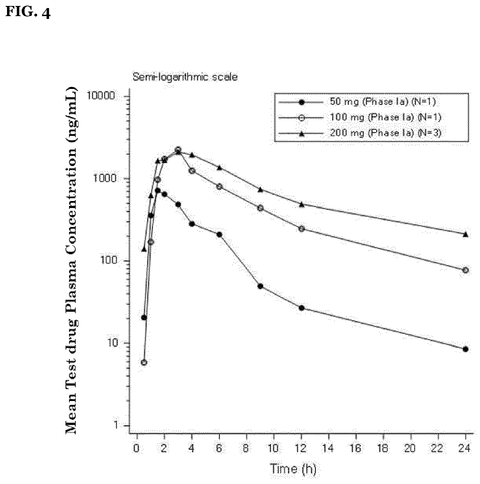

On the day 1 of cycle 1 (C1D1), plasma samples for pharmacokinetic analyses in clinical trial subjects were obtained before and after administration of the test drug (0.5, 1, 1.5, 2, 3, 4, 6, 9, 12 and 24 hours). As a result of the collected pharmacokinetic analyses, it was confirmed that a maximum plasma concentration after a single dose of 50 mg QD, 100 mg QD and 200 mg QD of the test drug ranged from to 2443 ng/ml, and a systemic exposure (based on AUC₀₋₂₄) ranged from 2760 to 21850 ng*h per mL. It was confirmed that the exposure concentration of the test drug increased in a dose-dependent manner in the dose range of 50 mg to 200 mg (FIG. 4).

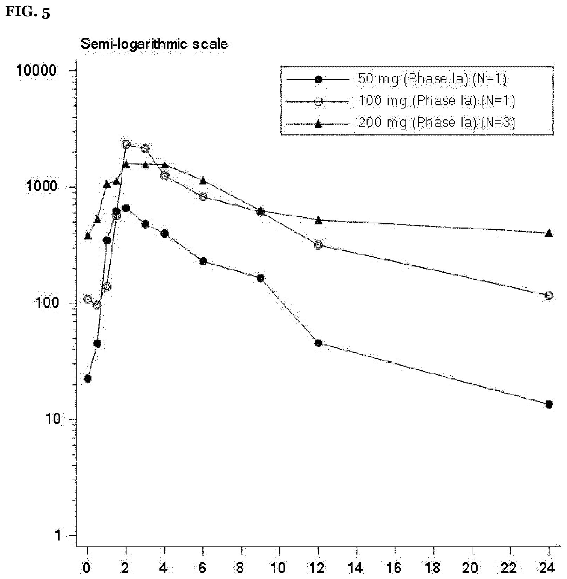

In addition, the mean plasma concentration values in the multiple dosing range of the 50 mg QD, 100 mg QD, and 200 mg QD cohorts showed the PK values from multiple dosing range of 50mg to 200mg cohort resulted in an approximate 2.92-fold and 4.97-fold increase in exposure based on the Cₘₐₓ and AUCₜₐᵤ, respectively. In addition, it was confirmed that the exposure concentration of the test drug increased in a dose-dependent manner in the dose range of 50 mg to 200 mg, and there was no accumulation observed in the dose range of 50 mg to 200 mg. (FIG. 5).

### 2) Pharmacodynamics

Blood samples for pharmacodynamic analyses of the test drug were collected in phase Ia (dose escalation part). Whole blood samples were evaluated by flow-cytometry after coimmunostaining for CD3+, CD4+, CD25+, CD127low and CD8+. Through this, we tried to observe the effects of the test drug on regulatory T cells (T_{regs}) and CD8+ T cells.

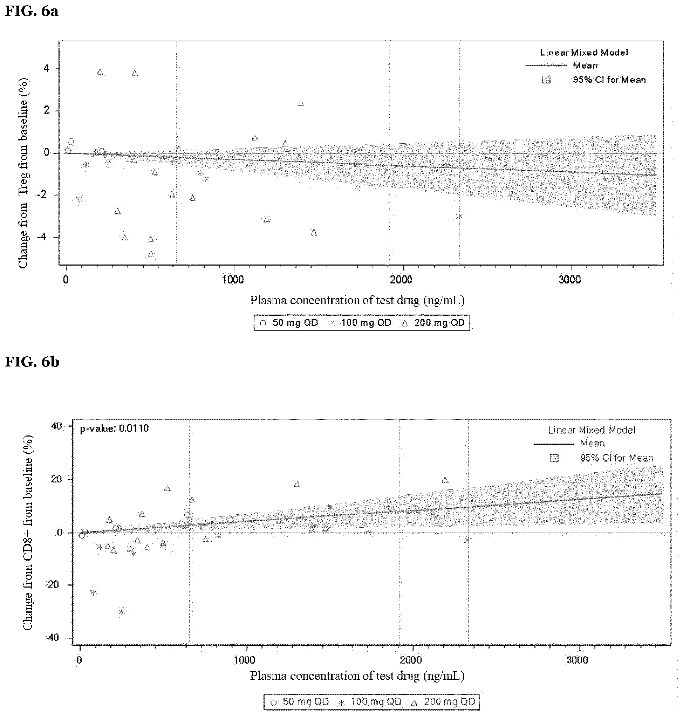

As a result, it was confirmed that as the plasma concentration of the test drug increased, regulatory T cells (T_{regs}) decreased and CD8+ T cells increased compared to the baseline value (FIGS. 6a and 6b). The above results confirm that the decrease in regulatory T cells (T_{regs}) and the increase in CD8+ T cells occur depending on the exposure of the test drug according to the pharmacokinetics, thereby confirming that the pharmacokinetics of the test drug and the pharmacodynamics are correlated with each other.

## Claims

1. A pharmaceutical composition for preventing or treating peripheral T-cell lymphoma (PTCL), comprising a compound represented by formula 1 below or pharmaceutically acceptable salts thereof in an amount of 25 mg or more and less than 325 mg as a compound represented by formula 1.

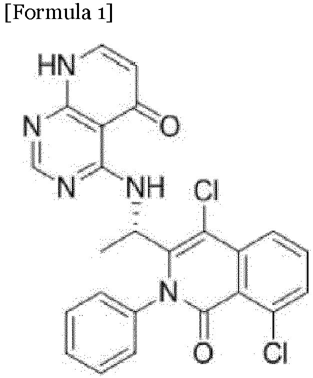

2. The pharmaceutical composition according to claim 1, wherein the composition comprises the compound represented by above formula 1 or pharmaceutically acceptable salts thereof in an amount of 200 mg or more and less than 325 mg as the compound represented by formula 1.

3. The pharmaceutical composition according to claim 1, wherein the composition comprises the compound represented by above formula 1 or pharmaceutically acceptable salts thereof in an amount of 200 mg as the compound represented by formula 1.

4. The pharmaceutical composition according to claim 1, wherein the composition is administered to a subject who has received at least one or more chemotherapies after being diagnosed with peripheral T-cell lymphoma.

5. The pharmaceutical composition according to any one of claims 1 to 4, wherein the composition is administered once daily for four weeks or more.

6. The pharmaceutical composition according to any one of claims 1 to 4, wherein the composition is orally administered.

7. The pharmaceutical composition according to any one of claims 1 to 4, wherein the composition decreases regulatory T cells (T_{regs}) and increases CD8+ T cells.
